# EUROPEAN PATENT SPECIFICATION

(11) **EP 0 731 720 B1**
(45) Date of publication and mention of the grant of the patent: **10.04.2002**
(21) Application number: 95902513.1
(22) Date of filing: 10.11.1994
(51) Int. Cl.: A61M 37/00

(54) **CARDIOPULMONARY BYPASS SYSTEM FOR CLOSED-CHEST INTERVENTION**
KARDIOPULMONALER BYPASS FÜR EINGRIFFE BEI GESCHLOSSENEM THORAX
SYSTEME DE CIRCULATION EXTRACORPORELLE POUR INTERVENTION A THORAX FERME

(30) Priority: 03.12.1993 US 162742
(43) Date of publication of application: 18.09.1996
(60) Divisional application: 01112373.4
(73) Proprietor: HEARTPORT, INC., Redwood City, California 94063 (US)
(72) Inventor: STEVENS, John, H., Palo Alto, CA 94303 (US); STERMAN, Wesley, D., San Francisco, CA 94109 (US); MACHOLD, Timothy, R., Moss Beach, CA 94038 (US); GIFFORD, Hanson, S., Woodside, CA 94062 (US)
(74) Representative: Harrison, David Christopher
(86) International application number: US9412986
(87) International publication number: WO9515192

(56) References cited:
- US-A- 5 011 469
- US-A- 5 195 942

## Description

This invention relates generally to devices and methods for performing cardiovascular, pulmonary, and neurosurgical procedures in which the patient is placed on cardiopulmonary bypass. More specifically, the invention relates to less-invasive devices and methods for establishing cardiopulmonary bypass and performing interventional procedures in the heart and great vessels.

Various cardiovascular, neurosurgical, pulmonary and other interventional procedures, including repair or replacement of aortic, mitral and other heart valves, repair of septal defects, pulmonary thrombectomy, coronary artery bypass grafting, angioplasty, atherectomy, treatment of aneurysms, electrophysiological mapping and ablation, and neurovascular procedures, are performed with the patient connected to cardiopulmonary bypass (CPB) equipment to maintain circulation of oxygenated blood throughout the patient's circulatory system. In some of these procedures, such as heart valve replacement and coronary artery bypass grafting, cardiac function is arrested, and peripheral circulation of oxygenated blood is maintained completely by a CPB system. In other procedures, such as angioplasty and atherectomy, the heart remains beating, and CPB is used to assist the heart in maintaining circulation of oxygenated blood during the procedure.

To establish cardiopulmonary bypass according to conventional techniques, a venous cannula is introduced into a major vein such as the inferior vena cava, or into the heart itself, to withdraw deoxygenated blood from the patient and deliver the deoxygenated blood to a CPB system for oxygenation. An arterial cannula is introduced into a major artery such as the aorta, an iliac artery, or a femoral artery, for delivering oxygenated blood from the CPB system to the patient's arterial system.

For endovascular procedures such as angioplasty and atherectomy in which cardiac function need not be arrested, interventional devices are introduced into an artery such as a femoral artery, and the devices are transluminally positioned at the treatment site where the procedure is performed. For example, in angioplasty or atherectomy, a catheter is introduced into a femoral artery and advanced through the aorta into a coronary artery to treat an occluded region therein. If CPB is utilized during such procedures, the arterial and venous CPB cannulae are usually introduced into a femoral artery and femoral vein, respectively, by means of a surgical cut-down in the groin area on one side of a patient's body. Interventional devices may then be introduced into a femoral artery in the groin area on the other side of the patient's body.

In those procedures in which cardiac function is arrested, on the other hand, the heart and coronary arteries must be isolated from the remainder of the patient's arterial system. Using conventional techniques, the sternum is cut longitudinally (a median sternotomy), providing access between opposing halves of the anterior portion of the rib cage to the heart and other thoracic vessels and organs. Alternatively, a lateral thoracotomy is formed, wherein an incision, typically 10 cm to 20 cm in length, is made between two ribs. A portion of one or more ribs may be permanently removed to optimize access. Through this large opening in the chest, a mechanical cross-clamp may be placed externally on the ascending aorta downstream of the ostia of the coronary arteries, but upstream of the brachiocephalic artery, so as to allow oxygenated blood from the CPB system to reach the arms, neck, head, and remainder of the body. A catheter is then introduced through the sternotomy or thoracotomy and inserted into the ascending aorta between the cross-clamp and the aortic valve. Cardioplegic fluid is infused through the catheter into the aortic root and coronary arteries to perfuse the myocardium. An additional catheter may be introduced into the coronary sinus for retrograde perfusion of the myocardium with cardioplegic fluid. In addition, the myocardium is usually cooled by irrigation with cold saline solution and/or application of ice or cold packs to the myocardial tissue. Cardiac contractions will then cease.

While such open-chest techniques can produce significant benefits for some patients, such techniques entail weeks of hospitalization and months of recuperation time, in addition to the pain and trauma suffered by the patient. Moreover, application of an external cross-clamp to a calcified or atheromatous aorta may cause the release of emboli into the brachiocephalic, carotid or subclavian arteries with serious consequences such as strokes.

US-A-5011469 (Buckberg et al) discloses an apparatus and method for cardiopulmonary bypass. A venous catheter is inserted into a femoral vein and an arterial cannula is inserted into a femoral artery. A pump between the venous catheter and the arterial cannula circulates the patient's blood. A vent catheter is passed through the arterial system to the left ventricle of the heart where it removes bronchial blood and allows the ventricle to rest.

In response to the problems outlined above, new techniques have been developed to facilitate the performance of cardiac procedures such as heart valve repair and replacement using endovascular instruments, eliminating the need for a thoracotomy as well as the need for an external aortic cross-clamp. Such procedures are described in US-A-5 370 695 (application Serial No. 07/730,559) which is assigned to the assignee of the present invention. Similarly, in commonly-assigned US-A-5 452 733 (application Serial No. 08/023,778), methods and devices are described for performing coronary artery bypass grafting and other procedures through small incisions or cannulae positioned through the chest wall, obviating the need for a thoracotomy. This new generation of minimally-invasive cardiac procedures provides significant advantages over conventional open surgical techniques, including reduced mortality and morbidity, decreased patient suffering, reduced hospitalization and recovery time, and lowered medical costs relative to open-chest procedures.

In order to arrest cardiac function according to the techniques described in the forementioned patent applications, once cardiopulmonary bypass (CPB) is established, an aortic occlusion catheter is transluminally positioned, preferably from a femoral artery, into the ascending aorta so that an expandable member on the distal end of the catheter is disposed between the coronary ostia and the brachiocephalic artery. The expandable member is expanded to occlude the ascending aorta, stopping the flow of blood therethrough. Cardiac function is then arrested, usually by delivering cardioplegic fluid through a lumen in the aortic occlusion catheter into the ascending aorta upstream of the expandable member, and/or by delivering cardioplegic fluid in a retrograde manner through a catheter positioned in the coronary sinus.

Using the closed-chest techniques described in the forementioned patent applications, the arterial and venous CPB cannulae are preferably introduced into a femoral artery and a femoral vein, respectively, and transluminally positioned in the desired arterial and venous locations. The aortic occlusion catheter for partitioning the ascending aorta and delivering cardioplegic fluid is preferably introduced into a femoral artery as well. In addition, a cardiac venting catheter is usually introduced through the internal jugular vein or femoral vein to remove blood from within the heart, usually from the pulmonary artery. Further, where retrograde infusion of cardioplegic fluid is utilized, a retroperfusion catheter is introduced through the internal jugular vein or femoral vein into the coronary sinus. Thus, for a closed-chest procedure utilizing cardiopulmonary bypass, endovascular aortic occlusion, pulmonary arterial venting, and retrograde perfusion of cardioplegia, three venous catheters and two arterial catheters are utilized, requiring the use of both femoral arteries, at least one femoral vein, and the internal jugular vein in the neck for introduction of these catheters.

In order to minimize trauma and the risk of complications such as infection, it is generally desirable to minimize the number of vascular penetrations or "sticks" which are made in a patient during a procedure. Such penetrations are a significant cause of morbidity and mortality in cardiac procedures. The risks are greater where the penetrations are either surgical cut-downs or large percutaneous penetrations, as are usually required for introduction of the forementioned aortic occlusion catheter and venous and arterial CPB cannulae. The risks are particularly high when such penetrations are made on arterial vessels.

Moreover, in some cases, one or more of a patient's femoral arteries, femoral veins, or other vessels for arterial and venous access may not be available for introduction of cannulae, due to inadequate vessel diameter, vessel stenosis, vascular injury, or other conditions. In such cases, there may not be sufficient arterial and venous access to permit the use of femoral arterial and venous CPB cannulae as well as other interventional devices, including an aortic occlusion catheter, a cardiac venting catheter, an angioplasty or atherectomy catheter, or other device introduced through a femoral vein or artery, contemporaneously as part of a single surgical procedure. Therefore, unless alternate arterial or venous access for one or more of these catheters can be found, the procedure cannot be performed using minimally-invasive techniques.

Improved methods and devices are therefore needed for establishing CPB and performing interventional procedures that reduce the number of arterial and venous penetrations required for CPB cannulae and other endovascular devices. The methods and devices will preferably facilitate isolating the heart and coronary arteries from the remainder of the arterial system, arresting cardiac function, and establishing cardiopulmonary bypass without the open-chest access provided by a thoracotomy. The methods and devices should minimize the number of arterial and venous penetrations required in such closed-chest procedures, and desirably, should require no more than one femoral arterial penetration and one femoral venous penetration. In addition to procedures requiring arrest of cardiac function, the methods and devices should be useful for a variety of closed-chest interventional procedures that require the use of cardiopulmonary bypass, even where cardiac function is not arrested.

The present invention provides an endovascular device as set out in claim 1 for establishing cardiopulmonary bypass and performing interventional procedures within the heart and great vessels with a minimum of arterial and venous penetrations. The device further facilitates partitioning a patient's ascending aorta between the coronary ostia and the brachiocephalic artery to isolate the heart and coronary arteries from the remainder of the arterial system, and arresting cardiac function, by means of an endovascular device introduced through a femoral or other artery.

Using the device of the invention, all blood flow through the ascending aorta may be blocked, cardioplegic fluid may be introduced through the coronary arteries to perfuse the myocardium, and oxygenated blood from a CPB system may be infused into the arterial system downstream from the point of aortic occlusion, all through a single femoral arterial penetration. Moreover, blood may be vented from the heart to prevent distension of the myocardium, and deoxygenated blood withdrawn from a venous location for oxygenation by the CPB system, all through a single femoral or jugular venous penetration.

With the patient connected to cardiopulmonary bypass equipment to maintain circulation of oxygenated blood while the heart is stopped, surgical procedures may be performed on the heart, coronary blood vessels and other body structures using thoracoscopic and/or endovascular tools, without the need for a conventional gross thoracotomy. Moreover, by partitioning the aorta by endovascular occlusion rather than by external cross-clamping, the device of the invention may substantially reduce the risk of embolus release associated with such cross-clamping.

For purposes of the present application, "downstream" means in the direction of normal blood flow through a blood vessel, i.e., further from the heart in the arterial system, and closer to the heart in the venous system. "Upstream" means in the direction opposite the downstream direction. With respect to devices, "proximal" means in the direction toward the end of the device that is closest to and held or manipulated by the user, while "distal" means in the direction away from the user, opposite the proximal direction.

Preferably, means are provided at the proximal end of the bypass cannula for fluidly connecting the blood flow lumen to a cardiopulmonary bypass (CPB) system. Interventional means are provided at the distal end of the catheter shaft for performing an interventional procedure in the heart or in a great vessel near the heart.

The interventional means comprises a device for partitioning the ascending aorta between the coronary ostia and the brachiocephalic artery. An expandable means such as an inflatable balloon is disposed at the distal end of the catheter shaft for occluding the ascending aorta between the coronary ostia and the brachiocephalic artery so as to block substantially all blood flow therethrough. Additionally, the device may include means at the proximal end of the catheter shaft for delivering cardioplegic fluid through the inner lumen of the catheter shaft into the patient's ascending aorta upstream of the occluding means. The bypass cannula is configured for introduction into an artery in the patient, and the blood flow lumen in the bypass cannula is connected to a means for delivering oxygenated blood into the patient's arterial system, such as a cardiopulmonary bypass system.

The catheter shaft may be fixed to the bypass cannula, and may be an integral part thereof, i.e., an extension from the distal end of the bypass cannula. In this configuration, the bypass cannula has a lumen, which may comprise the blood flow lumen, in fluid communication with the inner lumen in the catheter shaft. Alternatively, the catheter shaft is slidably disposed in the blood flow lumen of the bypass cannula, and may be removable from the bypass cannula, and/or limited in its movement relative to the bypass cannula. The bypass cannula may further be provided with a plurality of ports along a distal portion of its length in fluid communication with the blood flow lumen to enhance the flow of blood into or out of the blood flow lumen. The blood flow lumen is preferably configured to facilitate a fluid flow of at least about 4 liters/minute at a pressure of less than about 250 mmHg.

The bypass cannula may further have an adaptor assembly mounted to its proximal end. The adaptor assembly has first and second access ports in communication with the blood flow lumen, the first access port being configured to receive the catheter shaft, and the second access port being configured for connection to the oxygenated blood delivery means (in the arterial embodiment) or a means for receiving and oxygenating deoxygenated blood (in the venous embodiment). Usually, a hemostasis valve or other sealing means is mounted in the first access port to prevent leakage of blood therefrom, both when the catheter shaft is inserted through the first access port as well as when the catheter shaft is removed from the first access port.

In a preferred embodiment, the catheter shaft has a length of at least about 80 cm to facilitate transluminal positioning from a femoral artery into the heart or into a great vessel such as the ascending aorta near the heart. The bypass cannula will usually have a somewhat shorter length. The bypass cannula preferably has a length between about 10 cm and 60 cm, and more preferably about 15 cm to 30 cm, such that the outflow port at the distal end of the bypass cannula is disposed a substantial distance downstream of the occluding member on the catheter shaft.

Using the device of the invention, a patient's heart can be arrested and the patient placed on cardiopulmonary bypass without a conventional gross thoracotomy, thereby reducing mortality and morbidity, decreasing patient suffering, reducing hospitalization and recovery time, and lowering medical costs relative to previous open-chest procedures. The endovascular partitioning device of the invention permits blood flow through the ascending aorta to be completely blocked between the coronary ostia and the brachiocephalic artery in order to isolate the heart and coronary arteries from the remainder of the arterial system. This has significant advantages over the aortic cross-clamps used in current cardiac procedures, not only obviating the need for a gross thoracotomy, but providing the ability to stop blood flow through the aorta even when calcification or other complications would make the use of an external cross-clamp undesirable. Moreover, use of the device of the invention accomplish this with a minimum of arterial penetrations, thereby minimizing trauma and the risk of complications such as infection.

The device of the invention may further be useful to provide cardiopulmonary bypass during endovascular interventional procedures in which cardiac function may or may not be arrested. Such procedures may include angioplasty, atherectomy, heart valve repair and replacement, septal defect repair, treatment of aneurysms, myocardial mapping and ablation, myocardial drilling, and a variety of other procedures wherein endovascular interventional devices are introduced through the bypass cannula of the invention and advanced into the heart or great vessels. In this way, the invention facilitates cardiopulmonary bypass during such procedures without requiring additional arterial or venous penetrations.

A further understanding of the nature and advantages of the invention may be realized by reference to the remaining portions of the specification and the drawings.

Figure 1 is a side elevational view of an endovascular device for partitioning the ascending aorta between the coronary ostia and brachiocephalic artery constructed in accordance with the principles of the present invention.

Figure 1A is an end view of a distal portion of the device of Figure 1 illustrating the skew of the shaped distal portion.

Figures 1B and 1C are side elevational views showing alternative embodiments of the shaped distal portion of the device of Figure 1.

Figure 2A is a perspective view of a distal portion of the device of Figure 1 in a first embodiment thereof.

Figure 2B is a perspective view of a distal portion of the device of Figure 1 in a second embodiment thereof.

Figures 3 and 4 are transverse cross-sections taken along lines 3-3 and 4-4 in Figures 2A and 2B, respectively.

Figures 5A and 5B are transverse cross-sections taken along line 5-5 in Figure 2A, showing alternative embodiments of the shaft of the device illustrated therein.

Figure 6 is a transverse cross section taken along line 6-6 in Figure 2B.

Figure 7 is a front view of a portion of a patient's arterial system illustrating the introduction and advancement of the device of Figure 1 in the femoral artery, iliac artery and aorta.

Figure 8 schematically illustrates a system for arresting the heart wherein the device of Figure 1 is positioned in the ascending aorta with cardioplegic fluid delivery means connected to the proximal end and a cardiopulmonary bypass system connected to the patient.

Figure 9 illustrates the distal portion of the device of Figure 1 positioned in the ascending aorta with the occluding means expanded and a tissue cutting device extended from the distal end.

Figures 10A-10B are side and transverse cross-sections, respectively, of an endovascular partitioning device.

Figures 11A-11B are side elevational and transverse cross-sectional views, respectively, of an endovascular partitioning device.

Figure 12A is a side elevational view of an endovascular partitioning device.

Figure 12B is a transverse cross section taken along the line 12B-12B in Figure 12A, showing a shaping element positioned in an inner lumen in the shaft.

Figure 13A is a side elevational view of an endovascular partitioning device constructed in accordance with the principles of the present invention.

Figure 13B is a transverse cross-section taken through line 13B-13B in Figure 13A.

Figure 13C is a transverse cross-section taken through line 13C-13C in Figure 13A, showing a hemostasis valve with the aortic occlusion catheter removed from the blood flow lumen in the bypass cannula in the device of Figure 13A.

Figure 13D is a perspective view of an obturator and guidewire for use with the infusion tube in the device of Figure 13A.

Figure 13E is a side cross-sectional view of the partitioning device of Figure 13A.

Figure 14A is a perspective view of a cardiac venting device.

Figure 14B is a transverse cross-section taken through line 14B-14B in Figure 14A.

Figure 14C is a transverse cross-section taken through line 14C-14C in Figure 14A, showing the hemostasis valve with the venting catheter removed from blood flow lumen of the bypass cannula.

Figure 14D is a perspective view of an alternative configuration of a distal portion of the device of Figure 14A.

Figure 14E is a perspective view of an obturator to facilitate introduction of the device of Figure 14A.

Figure 14F is a side cross-sectional view of the cardiac venting device of Figure 14A.

Figure 15A is side elevational view of a cardiac venting device.

Figure 15B is a transverse cross-section taken through line 15B-15B in Figure 15A.

Figure 15C is a side elevational view of an alternative configuration of a distal portion of the device of Figure 15A.

Figure 15D is transverse cross-section taken through line 15D-15D in Figure 15C.

Figure 16 is a front partial cut-away view of a patient's body showing the positioning of the aortic partitioning device and cardiac venting device.

### DETAILED DESCRIPTION SPECIFIC EMBODIMENTS

The invention provides an endovascular device for partitioning the ascending aorta, which is useful in performing a variety of cardiovascular, pulmonary, neurosurgical, and other procedures. The invention is especially useful in conjunction with minimally-invasive cardiac procedures such as those described in US-A-5 370 695 and 5 452 733 which are assigned to the assignee of the present invention. Procedures with which the invention may find use include repair or replacement of aortic, mitral, and other heart valves, repair of septal defects, pulmonary thrombectomy, electrophysiological mapping and ablation, coronary artery bypass grafting, angioplasty, atherectomy, treatment of aneurysms, as well as neurovascular and neurosurgical procedures. The invention is particularly advantageous in that it allows the heart to be arrested and the patient to be placed on cardiopulmonary bypass using only endovascular devices, obviating the need for a thoracotomy or other large incision. Moreover, even in conventional open-chest procedures, the endovascular aortic partitioning device of the invention will frequently find use where an external cross-clamp would raise substantial risks of embolus release due to calcification or other aortic conditions.

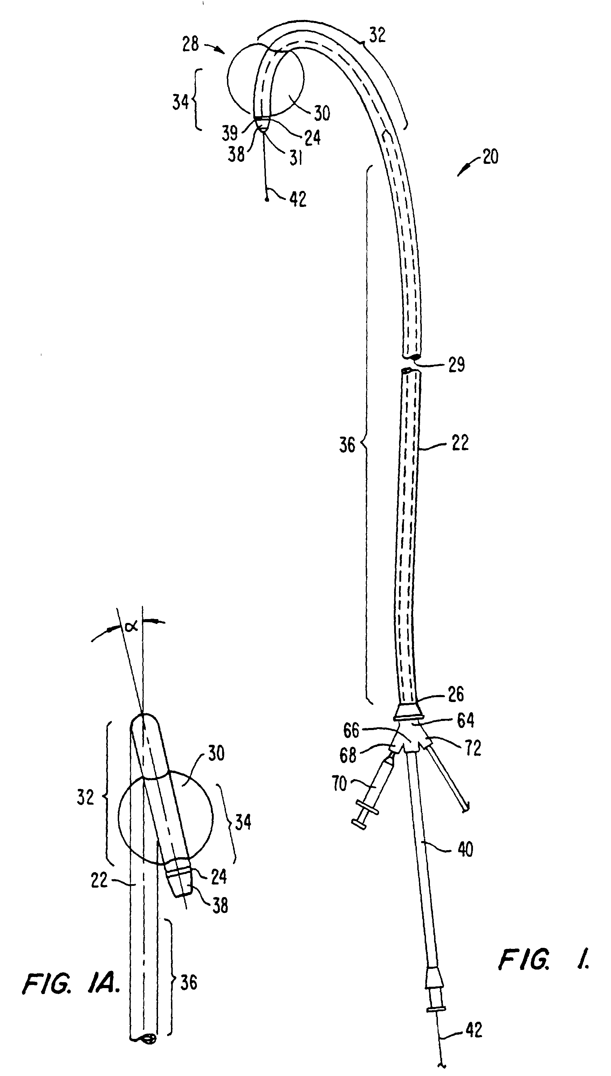

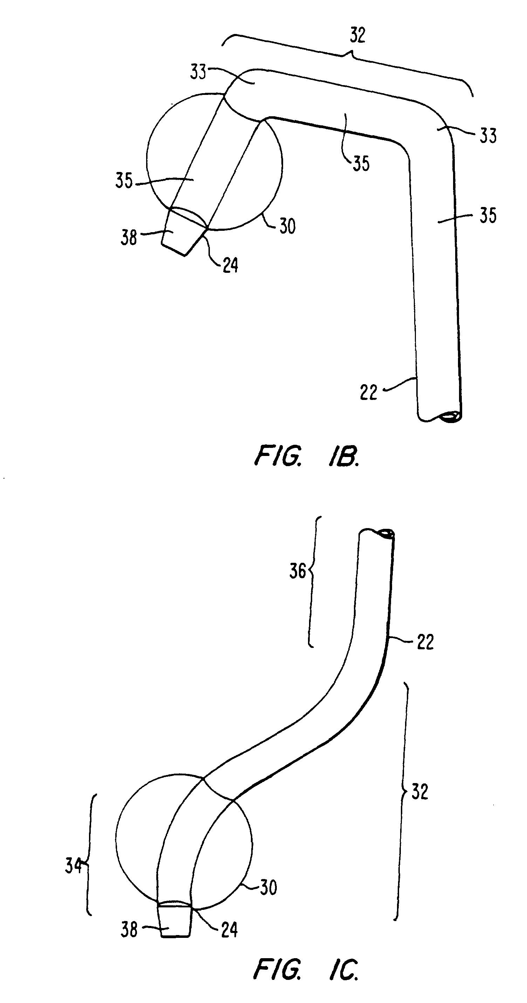

Turning now to the figures, a first preferred embodiment of an endovascular device for partitioning the ascending aorta according to the invention will be described. As illustrated in Figure 1, partitioning device 20 includes a shaft 22 having a distal end 24 and a proximal end 26. An expandable means 28 for occluding the ascending aorta is mounted to shaft 22 near distal end 24. In a preferred embodiment, occluding means 28 comprises a polymeric balloon 30 (shown inflated) of a material, geometry, and dimensions suitable for completely occluding the ascending aorta to block systolic and diastolic blood flow, as described more fully below.

Shaft 22 has a diameter suitable for introduction through a femoral or iliac artery, usually less than about 9 mm. The length of shaft 22 is preferably greater than about 80 cm, usually about 90-100 cm, so as to position balloon 30 in the ascending aorta between the coronary ostia and the brachiocephalic artery with proximal end 26 disposed outside of the body, preferably from the femoral or iliac artery in the groin area. Alternatively, the shaft may be configured for introduction through the carotid artery, through the brachial artery, or through a penetration in the aorta itself, wherein the shaft may have a length in the range of 20 to 60 cm.

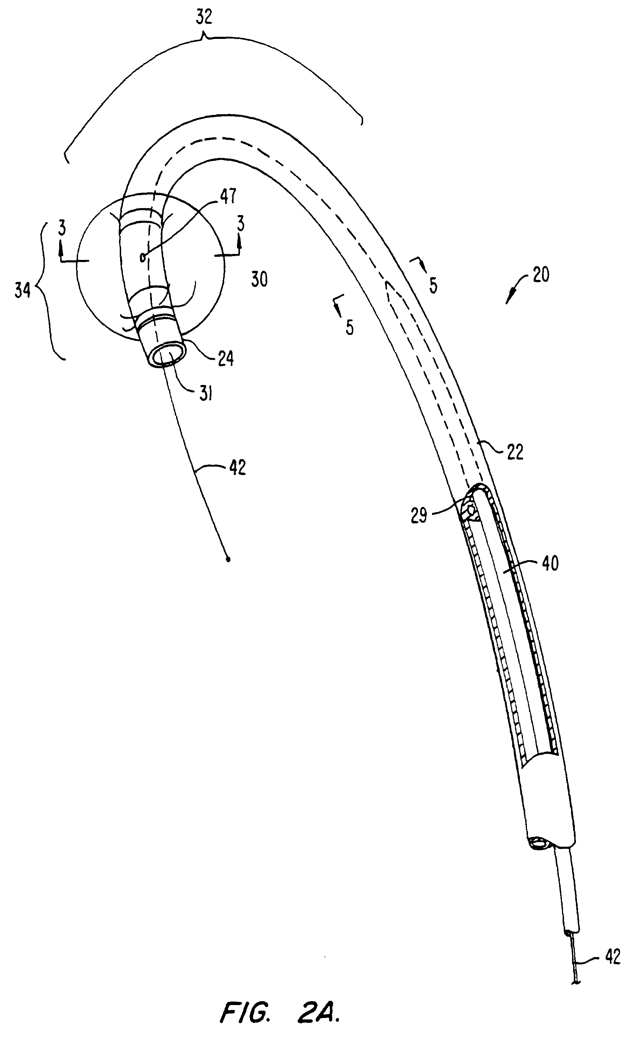

Partitioning device 20 further includes a first inner lumen 29, shown in Figures 2A-2B, extending between proximal end 26 and distal end 24 with an opening 31 at distal end 24. Additional openings in communication with inner lumen 29 may be provided on a lateral side of shaft 22 near distal end 24.

Shaft 22 has a shaped distal portion 32 configured to conform generally to the curvature of the aortic arch such that opening 31 at distal end 24 is spaced apart from the interior wall of the aorta and is axially aligned with the center of the aortic valve. Usually, shaped distal portion 32 will be generally U-shaped, such that a distal segment 34 is disposed at an angle between 135° and 225°, and preferably at approximately 180° relative to an axial direction defined by the generally straight proximal segment 36 of shaft 22. Shaped distal portion 32 will usually have a radius of curvature in the range of 20-80 mm (measured at the radial center of shaft 22), depending upon the size of the aorta in which the device is used. The configuration of shaped distal portion 32 allows distal segment 34 to be positioned centrally within the lumen of the ascending aorta and distal end 24 to be axially aligned with the center of the aortic valve, thereby facilitating infusion or aspiration of fluids as well as introduction of surgical tools through opening 31 without interference with the wall of the aorta, as described more fully below.

In an exemplary embodiment, shaped distal portion 32 is preshaped so as to maintain a permanent, generally U-shaped configuration in an unstressed condition. Such a preshaped configuration may be formed by positioning a mandrel having the desired shape in first inner lumen 29, then baking or otherwise heating shaft 22 and the mandrel for a sufficient time and sufficient temperature to create a permanent set therein, e.g., 1-3 hours at a temperature in a range of 120°C to 180°C, depending upon the material used for shaft 22.

Alternative embodiments of shaped distal portion 32 are illustrated in Figures 1B and 1C. In the embodiment of Figure 1B, U-shaped distal portion 32, rather than having a continuous, constant curvature, is preshaped in a more angular fashion, with bends 33 of relatively small curvature separating segments 35 which are either straight or of larger curvature. Bends 33 and/or segments 35 may further be configured to engage the inner wall of the aortic arch to deflect distal end 24 into a desired position in the ascending aorta.

In the embodiment of Figure 1C, shaped distal portion 32 is configured in a general "S" shape for introduction into the ascending aorta from a location superior to the aortic arch. In this way, distal segment 34 may be positioned within the ascending aorta, with proximal segment 36 extending from the aortic arch through the brachiocephalic artery to the carotid or brachial artery, or through a penetration in the aorta itself, to a point outside of the thoracic cavity.

As shown in Figure 1A, distal segment 34 may be skewed (non-coplanar) relative to a central longitudinal axis of proximal segment 36, in order to further conform to the shape of the patient's aortic arch and align with the center of the aortic valve. In an exemplary embodiment, distal segment 34 is disposed at an angle a relative to a plane containing the central axis of proximal portion 36, wherein a is between 2° and 30°, usually between 10° and 20°, and preferably about 15°. The shape and dimensions of shaped distal portion 32 and angle a of distal segment 34 may vary, however, according to the configuration of the aortic arch in any individual patient.

In a preferred embodiment, the device will include a soft tip 38 attached to distal end 24 to reduce the risk of damaging cardiac tissue, particularly the leaflets of the aortic valve, in the event the device contacts such tissue. Soft tip 38 may be straight or tapered in the distal direction, with an axial passage aligned with opening 31 at the distal end of shaft 22. Preferably, soft tip 38 will be a low durometer polymer such as polyurethane or Pebax, with a durometer in the range of 65 Shore A to 35 Shore D.

At least one radiopaque stripe or marker 39 is preferably provided on shaft 22 near distal end 24 to facilitate fluoroscopic visualization for positioning balloon 30 in the ascending aorta. Radiopaque marker 39 may comprise a band of platinum or other radiopaque material. Alternatively, a filler of barium or bismuth salt may be added to the polymer used for shaft 22 or soft tip 38 to provide radiopacity.

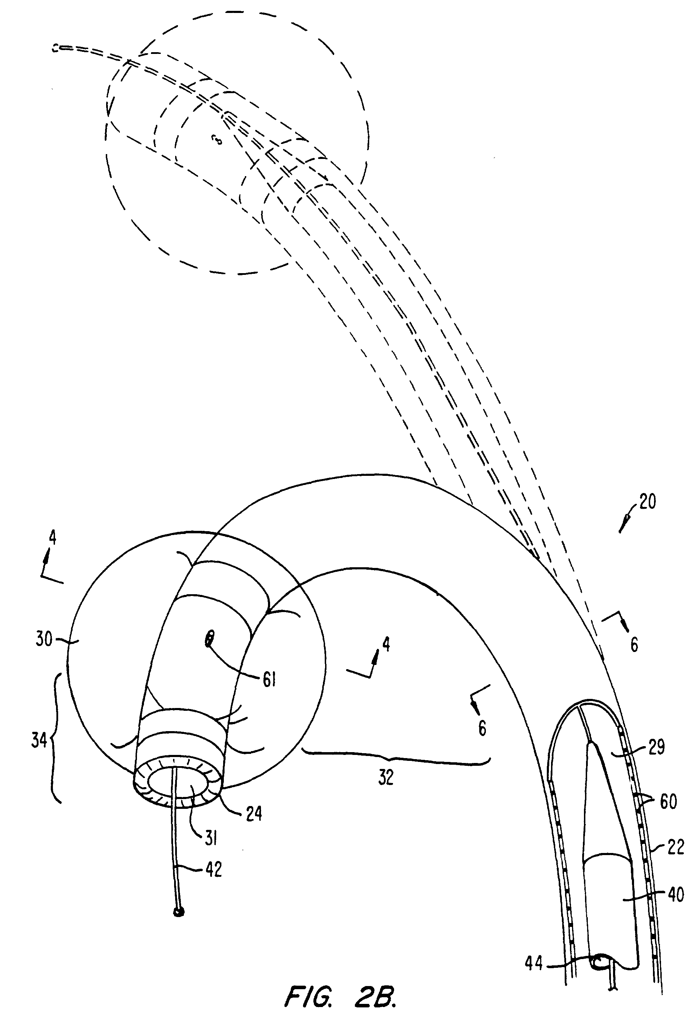

As illustrated in Figures 1, 2A and 2B, a straightening element 40 is disposed in first inner lumen 29 of shaft 22 so as to slide longitudinally relative to the shaft. Straightening element 40 may comprise a tubular stylet with a longitudinal passage 44 for receiving a guidewire 42 , as described below. Alternatively, element 40 may comprise a relatively stiff portion of the guidewire itself. Straightening element 40 may be a polymeric material or a biocompatible metal such as stainless steel or nickel titanium alloy with a bending stiffness greater than that of shaft 22. In this way, straightening element 40 may be advanced distally into preshaped distal portion 32 so as to straighten shaft 22, facilitating subcutaneous introduction of partitioning device 20 into an artery and advancement to the aortic arch. Straightening element 40 may then be retracted proximally relative to the shaft so that distal end 24 can be positioned in the ascending aorta with preshaped distal portion 32 conforming to the shape of the aortic arch.

A movable guidewire 42 is slidably disposed through first inner lumen 29, either through longitudinal passage 44 in straightening element 40 (Figure 2B), external and parallel to straightening element 40, or through a separate lumen (not shown) in shaft 22. Guidewire 42 extends through opening 31 in distal end 24 of shaft 22 and may be advanced into an artery distal to shaft 22, facilitating advancement of shaft 22 through the artery to the ascending aorta by sliding the shaft over the guidewire. In an exemplary embodiment, guidewire 42 is relatively stiff so as to at least partially straighten shaft 22, so that straightening element 40 is unnecessary for introduction of shaft 22. In this embodiment, guidewire 42 may be, for example, stainless steel or a nickel titanium alloy with a diameter of about 1.0 mm to 1.6 mm.

Shaft 22 may have any of a variety of configurations depending upon the particular procedure to be performed. In one embodiment, shaft 22 has a multi-lumen configuration with three non-coaxial parallel lumens in a single extrusion, as illustrated in Figures 2A, 3 and 5A. The three lumens include first inner lumen 29, which receives straightening element 40 and guidewire 42 and includes opening 31 at its distal end, an inflation lumen 46 which opens at an inflation orifice 47 near the distal end of shaft 22 in communication with the interior of balloon 30, and a third lumen 48 which has an opening (not shown) at distal end 24 of the shaft to sense pressure in the ascending aorta. In this embodiment, the largest transverse dimension of first inner lumen 29 is preferably about 1mm-4mm. Advantageously, the distal opening in third lumen 48 is radially offset from opening 31 in first inner lumen 29, so that infusion or aspiration of fluid through first inner lumen 29 will not affect pressure measurements taken through third lumen 48.

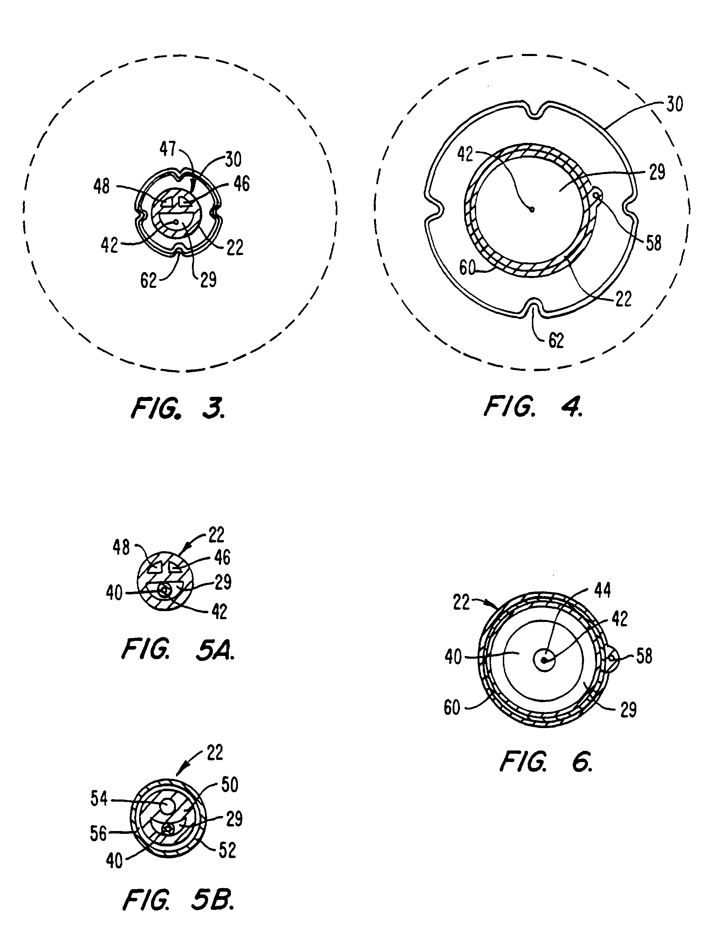

In a second embodiment, illustrated in Figure 5B, shaft 22 has a dual lumen inner member 50 and a coaxial outer member 52. Inner member 50 includes first inner lumen 29 which receives straightening element 40 and opens at distal opening 31, and a third lumen 54 which has an opening (not shown) at its distal end for measuring pressure in the ascending aorta. Outer member 52 defines a coaxial inflation lumen 56 which, at its distal end, is in communication with the interior of balloon 30. Balloon 30 and outer member 52 may comprise a single integrated extrusion, or balloon 30 may be bonded or otherwise attached to outer member 52 near the distal end of shaft 22 using well-known techniques. Outer member 52 may have an open distal end which communicates with the interior of balloon 30. Alternatively, the distal end of outer member 52 may be closed, for example, by bonding to the exterior of inner member 50, with an inflation orifice 47 provided as shown in Fig. 2A for communication between lumen 56 and the interior of the balloon.

In a third embodiment, illustrated in Figures 2B, 4 and 6, shaft 22 has a first inner lumen 29 of large diameter configured to receive various types of surgical instruments, as well as to receive straightening element 40. An inflation lumen 58 extends parallel to first inner lumen 29 and is in communication with the interior of balloon 30 through an inflation orifice 61, shown in Figure 2B. In this embodiment, shaft 22 may comprise a single extrusion containing inflation lumen 58 and inner lumen 29, or two individual tubes bonded to one another, one tube containing lumen 29 and the other containing inflation lumen 58. With this construction, shaft profile can be minimized while making lumen 29 as large as possible within the confines of the vessels in which the device is positioned. In this embodiment, first inner lumen 29 will have a diameter of at least about 5 mm and preferably about 8 mm. Partitioning device 20 thereby provides a passage of maximum diameter for endovascular introduction of surgical instruments such as visualization scopes, aspirators, irrigation tubes, cutting, stapling and suturing devices, and the like.

In some embodiments, as shown in Figures 2B, 4 and 6, a wire braid or coil 60 may be embedded in the wall of shaft 22 to enhance radial rigidity and to maintain the transverse dimensions of first inner lumen 29. It is particularly important to maintain the roundness of first inner lumen 29 where surgical tools are to be introduced through the first inner lumen. If shaft 22 is made of sufficient diameter to accommodate such tools through lumen 29, the shaft may tend to flatten or kink when advanced into the curved region of the aortic arch. The use of wire braid or coil 60 to maintain lumen roundness allows tool profile to be maximized and allows tools to be advanced through the lumen with minimum interference. Wire braid or coil 60 may be formed of stainless steel or other biocompatible material such as nickel titanium alloy, aramid fibers such as Kevlar™ (DuPont), or nylon.

Shaft 22 may be constructed of any of a variety of materials, including biocompatible polymers such as polyurethane, polyvinyl chloride, polyether block amide, or polyethylene. In a preferred embodiment of the device shown in Figure 2A, shaft 22 is urethane with a shore durometer in the range of 50D-80D. In the embodiment of Figure 2B, wherein shaft 22 may have a significantly larger diameter as well as an embedded coil which both increase stiffness, a polyurethane with shore durometer of 60A-100A may be used. Shaft 22 may have a bending modulus in the range of 70 to 100 kpsi, preferably about 80-90 kpsi. A bending modulus in this range provides sufficient stiffness to optimize pushability from a femoral or iliac artery to the ascending aorta, while providing sufficient flexibility to navigate the tortuous iliac artery and the aortic arch. Once partitioning device 20 has been positioned with distal end 24 in the ascending aorta, this bending modulus also facilitates exertion of a distally-directed force on shaft 22 from proximal end 26 to maintain the position of balloon 30 against the outflow of blood from the left ventricle as the balloon is inflated. In other embodiments, the dimensions, geometry and/or materials of shaft 22, as well as coil 60, may be varied over the length of the shaft so that the shaft exhibits variable bending stiffness in various regions. For example, preshaped distal portion 32 may be more flexible for tracking through the aortic arch, whereas proximal portion 36 may be stiffer for pushability and resistance to displacement.

Balloon 30 may be constructed of various materials and in various geometries. In a preferred embodiment, balloon 30 has a collapsed profile small enough for introduction into the femoral or iliac artery, e.g. 4-9 mm outside diameter, and an expanded (inflated) profile large enough to completely occlude the ascending aorta, e.g. 20-40 mm outside diameter. The ratio of expanded profile diameter to collapsed profile diameter will thus be between 2 and 10, and preferably between 5 and 10. The balloon is further configured to maximize contact of the working surface of the balloon with the aortic wall to resist displacement and to minimize leakage around the balloon, preferably having a working surface with an axial length in the range of about 3 to about 7 cm when the balloon is expanded. Textural features such as ribs, ridges or bumps may also be provided on the balloon working surface for increased frictional effects to further resist displacement.

Balloon 30 preferably has some degree of radial expansion or elongation so that a single balloon size may be used for aortas of various diameters. Materials which may be used for balloon 30 include polyurethanes, polyethylene terephthalate (PET), polyvinyl chloride (PVC), latex, ethylene vinyl acetate (EVA) and the like. However, balloon 30 must have sufficient structural integrity when inflated to maintain its general shape and position relative to shaft 22 under the systolic pressure of blood flow through the ascending aorta. In an exemplary embodiment, balloon 30 is constructed of polyurethane or a blend of polyurethane and polyvinyl such as PVC or EVA. It has been found that such materials have sufficient elastic elongation to accommodate a range of vessel diameters, while having sufficient structural integrity to maintain their shape and position in the ascending aorta when subject to outflow of blood from the left ventricle.

In a preferred embodiment, balloon 30 is further provided with a plurality of folds or pleats 62, shown in Figures 3 and 4, which allow the balloon to be collapsed by evacuation to a small collapsed profile for introduction into a femoral or iliac artery. In this embodiment, balloon 30 has a blow-up ratio, defined as the ratio of the fully-inflated outside diameter to the deflated outside diameter (before collapsing), of about 200%-400%, preferably 300%-400%. Pleats 62 are preferably at least three in number and each have a width representing approximately 5-25% of the circumference of the balloon when deflated (but not collapsed by subjecting the interior of the balloon to a vacuum). Pleats 62 may be formed into the balloon during the balloon-making process by using a dipping mandrel having longitudinal flutes formed in its periphery. The mandrel is dipped into a container of liquefied balloon material (e.g. polyurethane) so that a tubular layer of material solidifies onto the mandrel, conforming to the shape of the flutes. The mandrel is then removed, producing a pleated balloon of substantially constant thickness. Where a folded, rather than pleated, balloon is used, the folds may be formed after the balloon is made by vacuum collapsing the balloon onto a mandrel into the desired collapsed profile and heating the balloon, or by expanding the balloon under pressure and heat in a corrugated mold.

In alternative embodiments, occluding means 28 may comprise any of a variety of structures, including pivot, umbrella or fan-type occlusion mechanisms actuated by pull wire, torque cable, or other type of mechanical, hydraulic, electric, or shape-memory actuator. Further, occlusion means 28 may comprise multiple occlusion devices arranged in tandem on shaft 22; for example, a pair of balloons may be arranged one behind the other at the distal end of the shaft. In one embodiment, an occluding balloon is disposed on the shaft to be positionable in the ascending aorta, while a seating balloon is disposed distal to the occluding balloon so as to be positionable in the left ventricle through the aortic valve. By inflating the seating balloon in the left ventricle, the position of the occluding balloon in the ascending aorta may be maintained against the outflow of blood from the left ventricle.

Referring again to Figure 1, a triple-arm adaptor 64 is attached to the proximal end 26 of shaft 22. Triple-arm adaptor 64 includes a working port 66 in communication with first inner lumen 29 through which straightening element 40, guidewire 42, and in some embodiments, surgical or diagnostic instruments may be introduced, as described below. Working port 66 may also be adapted for infusion of fluid such as cardioplegic fluid, saline or contrast solution, as well as for aspiration of blood, fluids and debris through first inner lumen 29. Triple-arm adaptor 64 further includes an inflation port 68 in communication with the inflation lumen and configured for connection to an inflation fluid delivery device such as a syringe 70. A pressure measurement port 72 is in communication with the third lumen (48 or 54) and is adapted for connection to a pressure measurement device. Alternatively, where shaft 22 includes only first inner lumen 29 and inflation lumen 58 as in Figures 2B, 4 and 6, port 72 may be in communication with first inner lumen 29 and configured for pressure measurement, fluid infusion or aspiration.

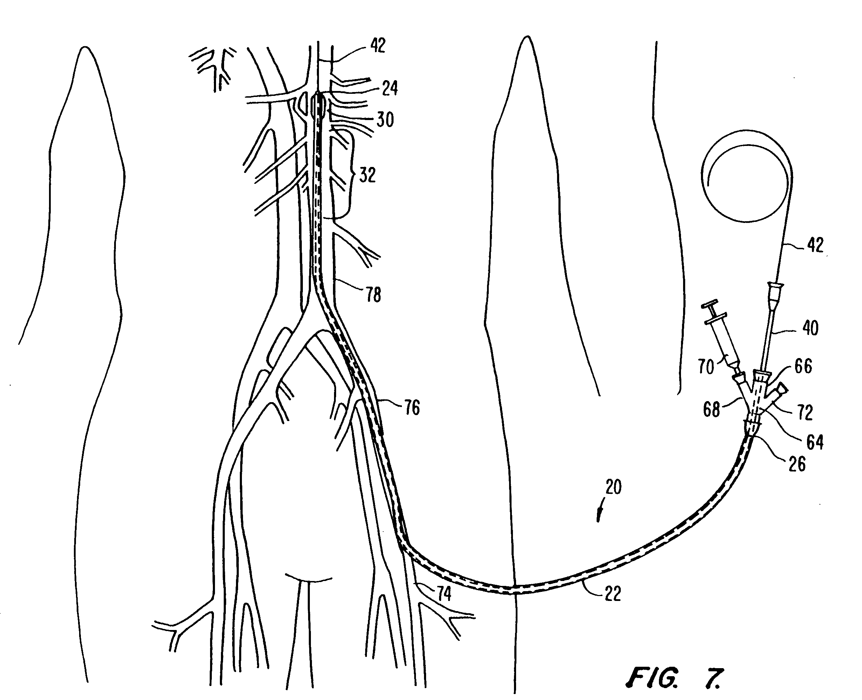

Referring now to Figures 7-9, a preferred use of the invention will be described. Initially, a partitioning device 20 of a size and configuration suitable for the particular patient must be selected. Usually, the patient's aorta will be observed by means of a fluoroscopic imaging to determine its size and shape, particularly in the region of the aortic arch. A partitioning device 20 will be selected having a length sufficient to allow occluding means 28 to be advanced into the ascending aorta from the point of introduction, which will preferably be a femoral or iliac artery in the groin area. Further, a partitioning device will be selected which has a preshaped distal portion 32 with dimensions and shape suitable for positioning the distal portion in the patient's aortic arch such that distal end 24 is spaced apart from the inner wall of the ascending aorta, preferably aligned with the center of the aortic arch. Usually, the preshaped distal portion will have a radius of curvature approximately equal to that of the aortic arch as measured to the center of the aorta, preferably within a tolerance of about +/- 10 mm.

Referring to Figure 7, partitioning device 20 is preferably subcutaneously inserted into a femoral or iliac artery 74 in the groin area using known techniques such as a cut-down or a percutaneous technique such as the Seldinger technique. Guidewire 42 is first introduced into femoral artery 74 and advanced toward the heart through iliac artery 76 and aorta 78 so that the distal end of guidewire 42 is in the ascending aorta (not shown in Figure 7). Straightening element 40 is inserted into lumen 29 of shaft 22 and positioned in preshaped distal portion 32 so as to straighten the preshaped distal portion. With balloon 30 deflated, shaft 22 is positioned over guidewire 42, introduced into femoral artery 74 and advanced over guidewire 42 through iliac artery 76 and aorta 78. A fluoroscope may be used for visualization of radiopaque markers 39 on shaft 22 to facilitate positioning. As an alternative or supplement to fluoroscopic imaging, ultrasonic echocardiography may be used by, for example, positioning an echocardiographic transducer in the esophagus.

As an alternative to femoral or iliac introduction, shaft 22 may be introduced into carotid artery 87 or brachial artery 89. In such cases, distal portion 32 of shaft 22 will usually have a generally S-shaped configuration, as described above with reference to Figure 1C. Such an S-shaped configuration facilitates positioning balloon 30 in the ascending aorta with shaft 22 extending superiorly from the aortic arch through brachiocephalic artery 86.

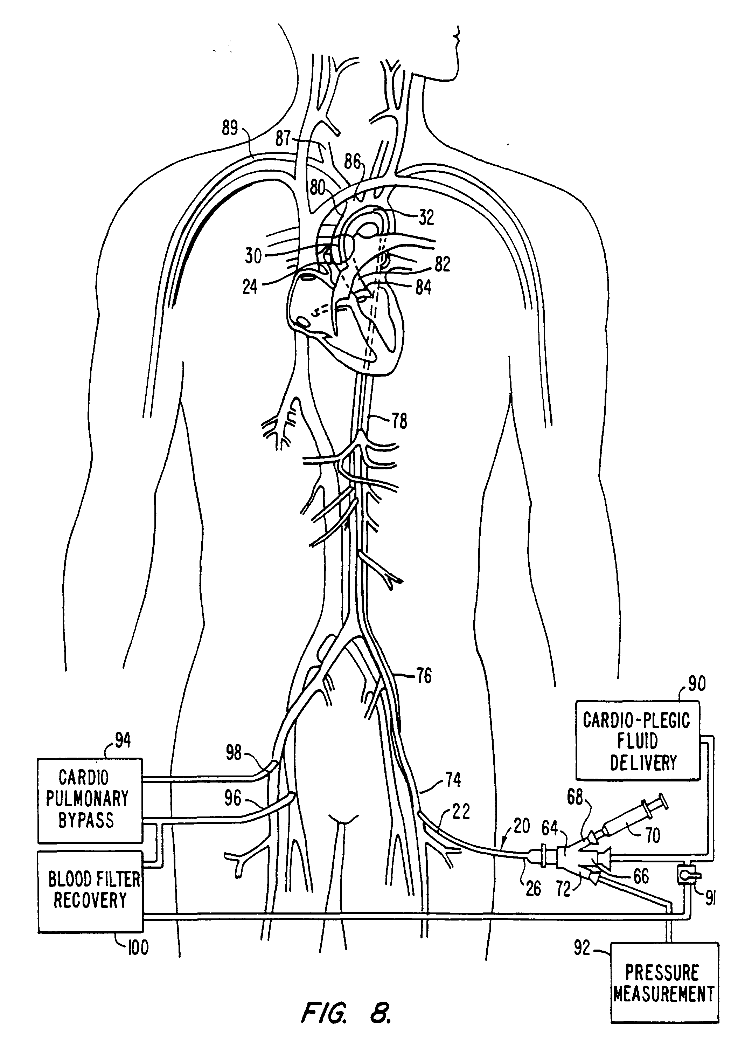

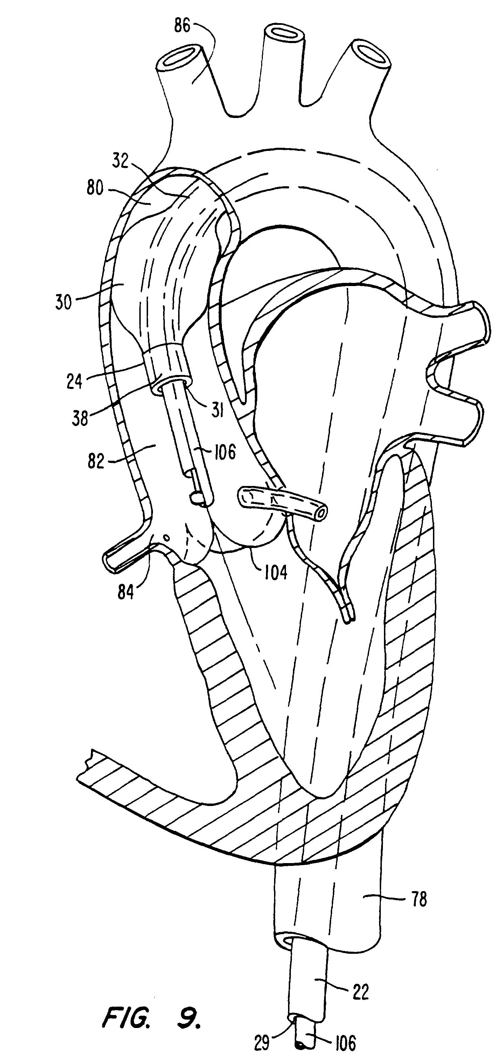

As illustrated in Figures 8 and 9, shaft 22 is advanced through aortic arch 80 until balloon 30 resides in ascending aorta 82 between coronary ostia 84 and brachiocephalic artery 86. As distal end 24 is advanced around the aortic arch, straightening element 40 is drawn proximally relative to shaft 22 so as to allow preshaped distal portion 32 to conform to the shape of the arch. In an alternative embodiment, a relatively stiff guidewire may be used without a separate straightening element, in which case the guidewire may remain in place as shaft 22 is advanced into the ascending aorta. Straightening element 40 and guidewire 42 may then be removed from shaft 22.

In an alternative technique, partitioning device 20 may be introduced into the aorta thoracoscopically. In this embodiment, distal end 24 of shaft 22 may be introduced through a small incision or cannula into the chest cavity. A small penetration is made in the aorta, either in the descending region or in the aortic arch. Shaft 22 is then inserted into the aorta using forceps or other thoracoscopic instruments introduced into the chest cavity through small incisions or cannulae. Such a technique may be useful where a patient's femoral or iliac arteries are unsuitable for introducing partitioning device 20 percutaneously or by cut down into those vessels.

As illustrated in Figure 8, once shaft 22 has been positioned so that balloon 30 is in ascending aorta 82 between coronary ostia 84 and brachiocephalic artery 86, balloon 30 is expanded by injecting an inflation fluid, usually a saline solution with a radiographic contrast agent, from syringe 70 through inflation port 68. In an exemplary embodiment, the balloon will be fully inflated in approximately 5-15 seconds, depending upon the size of the inflation lumen and the viscosity of the inflation fluid used. In some embodiments, blood may be allowed to flow through inner lumen 29 and directed to cardiopulmonary bypass system 94 (described below), thereby reducing the pressure of blood flow against balloon 30 during inflation. When fully inflated, the exterior surface of balloon 30 contacts the inner walls of the ascending aorta so as to fully occlude the vessel and block substantially all systolic and diastolic blood flow past the balloon. While the heart remains beating, blood may flow from the left ventricle through the aortic valve and into the coronary ostia so as to perfuse the myocardium through the coronary arteries. The heart and coronary arteries are thus isolated from the remainder of the arterial system.

In an alternative embodiment, a gaseous inflation fluid may be used in order to increase inflation speed. In this way, balloon 30 can be fully inflated in less time than the period between systolic pulses, reducing the likelihood that the outflow of blood from the left ventricle during systole will displace balloon 30 from its position in the ascending aorta. Preferably, helium is used as the inflation fluid, since helium, being highly soluble in blood, is unlikely to produce potentially injurious gas emboli in the event of leakage from the balloon. Alternatively, carbon dioxide may be used. A gas inflation pump and control device similar to those described in US-A-4,771,765 and 4,902,272, may be utilized for delivery of pressurized helium through inflation port 68. The inflation pump may be timed with the contractions of the heart to facilitate inflation of the balloon between systolic pulses. Using such a pump, balloon 30 may be fully inflated in less than about 1 second, and preferably less than about 0.5 second.

Figure 8 illustrates the components of a system for arresting the heart. A cardioplegic fluid delivery device 90 is connected to working port 66. A pressure measurement device 92 may be connected to port 72 to monitor pressure in the ascending aorta upstream of balloon 30 through first inner lumen 29 (or through an independent third lumen in shaft 22). The patient is placed on a cardiopulmonary bypass (CPB) system 94 to maintain circulation of oxygenated blood throughout the body. Usually, a venous cannula 96 is positioned in a femoral vein for withdrawing de-oxygenated blood. In addition, a pulmonary artery venting catheter (not shown) may be positioned through the right internal jugular vein into the pulmonary trunk to withdraw the blood contained therein, thereby decompressing the left atrium. The withdrawn blood is delivered to CPB system 94 which removes carbon dioxide and oxygenates the blood. The oxygenated blood is then delivered to a femoral or iliac artery via an arterial cannula 98. A blood filter and recovery system 100 may also be connected to port 66 in partitioning device 20 via a routing switch 101 to receive blood and other fluids and debris from first inner lumen 29 before or after delivery of cardioplegic fluid, filter the blood to remove impurities, and deliver the blood to CPB system 94 for return to the patient's circulatory system. Further aspects of a CPB system suitable for use in the system of the invention are described in F. Rossi et al., *Long-Term Cardiopulmonary Bypass By Peripheral Cannulation In A Model of Total Heart Failure,* Journal of Thoracic and Cardiovascular Surgery (1990), 100:914-921; U.S. Patent No. 4,540,399; and U.S. Patent No. 5,011,469.

With CPB established and balloon 30 blocking blood flow through the ascending aorta, the myocardium may then be paralyzed. In a preferred embodiment, a cardioplegic fluid such as potassium chloride (KCl) is delivered by delivery device 90 through working port 66. Preferably, delivery device 90 includes a cooler (not shown) which cools the cardioplegic fluid so as to maintain the heart at a low temperature, e.g. 5-10°C, and to minimize demand for oxygen. This is usually accomplished without applying external cooling to the heart as is applied in conventional open cardiac procedures. The cardioplegic fluid is infused into the ascending aorta through opening 31 at the distal end of partitioning device 20. The cardioplegic fluid flows through coronary ostia 84 into the coronary arteries so as to perfuse the myocardium. Cardioplegic fluid may also be infused in a retrograde manner through the coronary sinus, by means of a catheter (not shown) positioned transluminally through the right interior jugular vein. Heart contractions will then cease, with circulation to the remainder of the patient's body maintained by CPB system 94. Cardioplegic fluid flow to the patient's myocardium is maintained on a periodic basis, e.g., about every 20 minutes, so long as the myocardium is to remain paralyzed.

In addition to or instead of infusion of KCl, other techniques may be used to arrest heart contractions. The patient's body may be cooled in a cold-temperature environment or by application of cold-packs to the chest to reduce the temperature of the myocardium sufficiently to induce fibrillation. The myocardium may be cooled directly by infusion of cold fluid such as saline through the coronary arteries. Alternatively, electrical fibrillation may be accomplished by delivering electrical signals to the myocardium by means of electrodes placed on the exterior surface of the heart or externally on the chest. However, cardiac arrest by means of fibrillation is generally less desirable than chemical cardioplegic paralysis because there remains some degree of heart motion which could make surgical intervention more difficult and because there is a significantly higher demand for oxygen, reducing the safety and duration of the procedure.

Once the heart has been arrested and CPB established, a surgical procedure may be performed. The procedure will preferably be a less-invasive procedure performed endovascularly or thoracoscopically, as described in US-A-5 452 733. Surgical procedures which may be performed using the device and system of the invention include repair or replacement of the aortic, mitral and other heart valves, repair of ventricular and atrial septal defects, septal myotomy, cardiac mapping and ablation to correct arrhythmias, coronary artery bypass grafting, angioplasty, atherectomy, as well as pulmonary, neurosurgical, and other procedures.

Partitioning device 20 of the present invention is particularly advantageous for endovascular introduction of surgical instruments through the aorta for procedures such as heart valve repair and replacement. As illustrated in Figure 9, preshaped distal portion 32 of shaft 22 conforms to the shape of aortic arch 80 so that opening 31 at the distal end is positioned centrally within the ascending aorta and axially aligned with the center of aortic valve 104. This not only enhances infusion of cardioplegic fluid through opening 31, but ensures that surgical instruments such as valve cutter 106 introduced through first inner lumen 29 will be aligned with aortic valve 104, either to remove the valve, or to pass through it for intracardiac procedures. Advantageously, soft tip 38 at the distal end of shaft 22 prevents damage to tissue, particularly the fragile aortic valve leaflets, in the event of contact therewith.

While being particularly useful in conjunction with minimally-invasive cardiac procedures performed endovascularly and/or thoracoscopically, the partitioning device and system for arresting the heart disclosed herein are also useful in conventional open procedures performed with a thoracotomy. Partitioning device 20 may be used where an aortic cross-clamp would pose risks of embolus release due to calcification or other aortic conditions. In open procedures, partitioning device 20 may be introduced through the femoral or iliac arteries as described above, through the carotid artery 87, through the brachial artery 89, or through a penetration in the aorta itself, which is accessible as a result of the thoracotomy. In such cases, shaft 22 of partitioning device 20 may be substantially shorter in length, for example, 20 to 60 cm.

When the procedure has been completed, the heart is restarted by discontinuing any flow of cardioplegic fluid through partitioning device 20 or retrogradely through the coronary sinus, ventilating the lungs, and perfusing the coronary arteries with warm blood. The region upstream of balloon 30 may be irrigated by infusing a saline solution through first inner lumen 29. Blood and other fluids upstream of balloon 30 may then be aspirated through first inner lumen 29 to remove thrombi or other emboli which may have been produced during the procedure, preventing such emboli from entering the brachiocephalic, carotid, or subclavian arteries and greatly reducing the risk of complications such as strokes. Balloon 30 is deflated to allow normal flow of warm blood through the ascending aorta to the remainder of the arterial system. Normal heart contractions may resume promptly, or, if necessary, electrical defibrillation may be administered to correct heart rhythm. CPB is gradually discontinued, and CPB venous cannula 96 and arterial cannula 98 are removed. Partitioning device 20 is withdrawn from the body back through the site of entry, and the arterial penetration is closed. If the patient has been put under general anesthesia, the patient is then brought from anesthesia to consciousness.

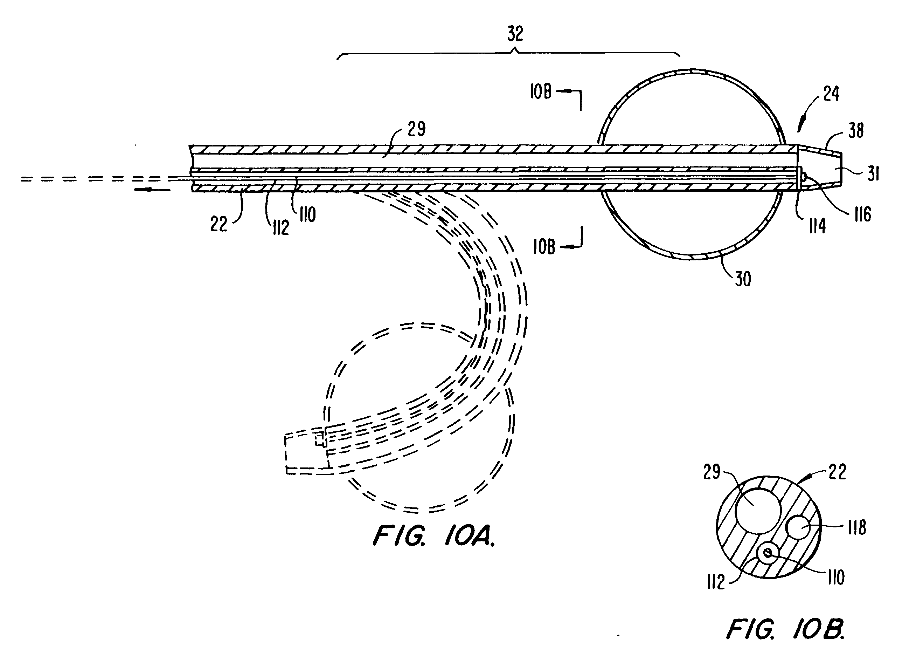

It will be understood by those of skill in the art that various alternative configurations of endovascular partitioning device 20 are possible without departing from the scope of the present invention. One such alternative embodiment is illustrated in Figures 10A-10B. In this embodiment, partitioning device 20 has a pull wire 110 disposed in a lumen 112 in shaft 22. Pull wire 110 is attached at its distal end to an anchor plate 114 at distal end 24 of shaft 22, preferably offset from the central longitudinal axis of shaft 22. In one embodiment, pull wire 110 extends through a hole in anchor plate 114 and is retained against the anchor plate by a ball 116 fixed to the distal end of pull wire 110. In other respects, device 20 is configured as described above in connection with Figures 1-9, including a balloon 30 mounted to shaft 22 near distal end 24, an inflation lumen 118 in communication with the interior of balloon 30, a soft tip 38 attached to distal end 24 of shaft 22, and an inner lumen 29 in communication with distal opening 31. Tension may be applied to the proximal end (not shown) of pull wire 110 to deflect the distal portion 32 of shaft 22 into a shape suitable for positioning distal portion 32 in the aortic arch (as shown in phantom in Figure 10A). In an alternative embodiment, an axially rigid, laterally-deflectable rod may be used in place of pull wire 110, whereby distal end 24 is deflected by applying a compressive force to the rod.

In an undeflected configuration (with tension relaxed on pull wire 110), distal portion 32 of the shaft is generally straight. Alternatively, all or part of distal portion 32 may be curved in an undeflected configuration to enhance positionability in the aortic arch. Preferably, a mechanism (not shown) will be provided at the proximal end of shaft 22 for applying tension to pull wire 110 and for locking the pull wire to maintain distal portion 32 in a desired shape. Various mechanisms may be used, such as those described in US-A-5,030,204. Usually, shaft 22 is introduced into an artery in a generally straight configuration, and tension is applied to pull wire 110 to deflect distal portion 32 as the shaft is advanced into the aortic arch. Once distal portion 32 is positioned in the aortic arch, tension on pull wire 110 is adjusted so as to position distal end 24 radially within the ascending aorta so as to be spaced apart from the inner wall of the aorta and axially aligned with the center of the aortic valve. Pull wire 110 is then locked in tension to maintain distal portion 32 in its deflected configuration.

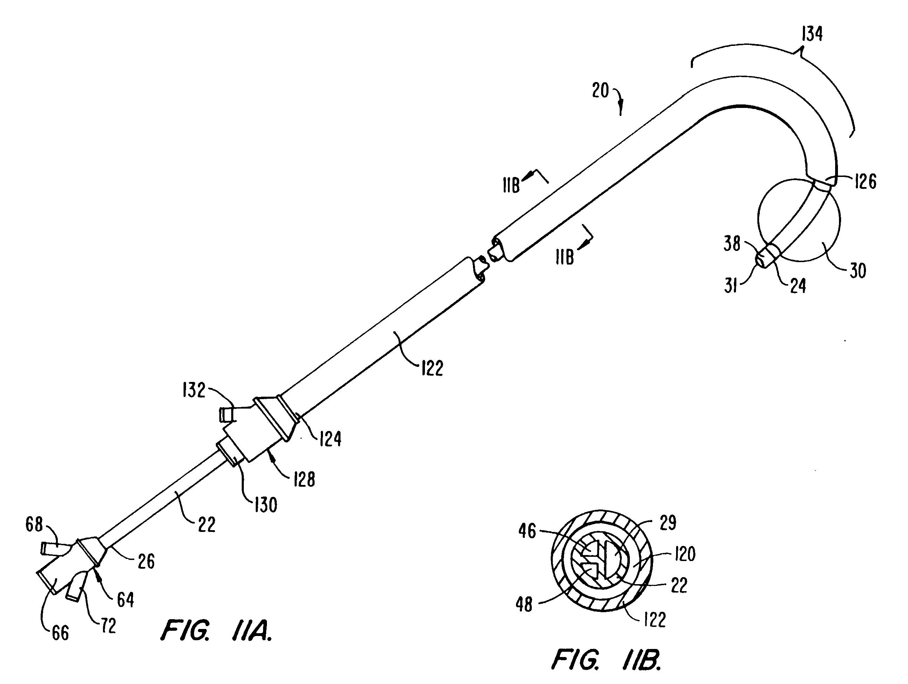

A further alternative embodiment of partitioning device 20 is illustrated in Figures 11A-11B. In this embodiment, shaft 22 is positionable in an interior lumen 120 of a guiding catheter 122. Device 20 may be configured as described above with reference to Figures 1-6, including balloon 30 near distal end 24, inner lumen 29, inflation lumen 46, pressure lumen 48, soft tip 38 attached to distal end 24, and triple-arm adaptor 64 attached to proximal end 26. Guiding catheter 122 has a proximal end 124 and a distal end 126, with axial lumen 120 extending therebetween. A soft tip (not shown) may be attached to distal end 126 to minimize injury to the aorta or aortic valve in the event of contact therewith. A proximal adaptor 128 is attached to proximal end 124, and has a first port 130 in communication with lumen 120 through which shaft 22 may be introduced, and a second port 132 in communication with lumen 120 for infusing or aspirating fluid. Port 130 may further include a hemostasis valve. Guiding catheter 122 also has a distal portion 134 which is either preshaped or deflectable into a shape generally conforming to the shape of the aortic arch. Techniques suitable for preshaping or deflecting distal portion 134 of guiding catheter 122 are described above in connection with Figures 1-6 and 10A-10B. In an exemplary embodiment, guiding catheter 122 is preshaped in a generally U-shaped configuration, with a radius of curvature in the range of 20-80 mm. In this embodiment, a stylet (not shown) like that described above in connection with Figures 1-6 is provided for straightening distal portion 134 for purposes of subcutaneously introducing guiding catheter 122 into an artery.

In use, guiding catheter 122 is introduced into an artery, e.g. a femoral or iliac artery, and advanced toward the heart until distal end 126 is in the ascending aorta. A guidewire (not shown) may be used to enhance tracking. Where a stylet is used to straighten a preshaped guiding catheter for subcutaneous introduction, the stylet is withdrawn as preshaped distal portion 134 is advanced through the aortic arch. Once guiding catheter 122 is in position, shaft 22 may be introduced through port 130 and lumen 120 and advanced toward the heart until balloon 30 is disposed between the coronary ostia and the brachiocephalic artery, distal to the distal end 126 of guiding catheter 122. The distal portion 32 of shaft 22 (Figure 1) is shaped to conform to the aortic arch by preshaped portion 134 of guiding catheter 122. Balloon 30 is then inflated to fully occlude the ascending aorta and block blood flow therethrough.

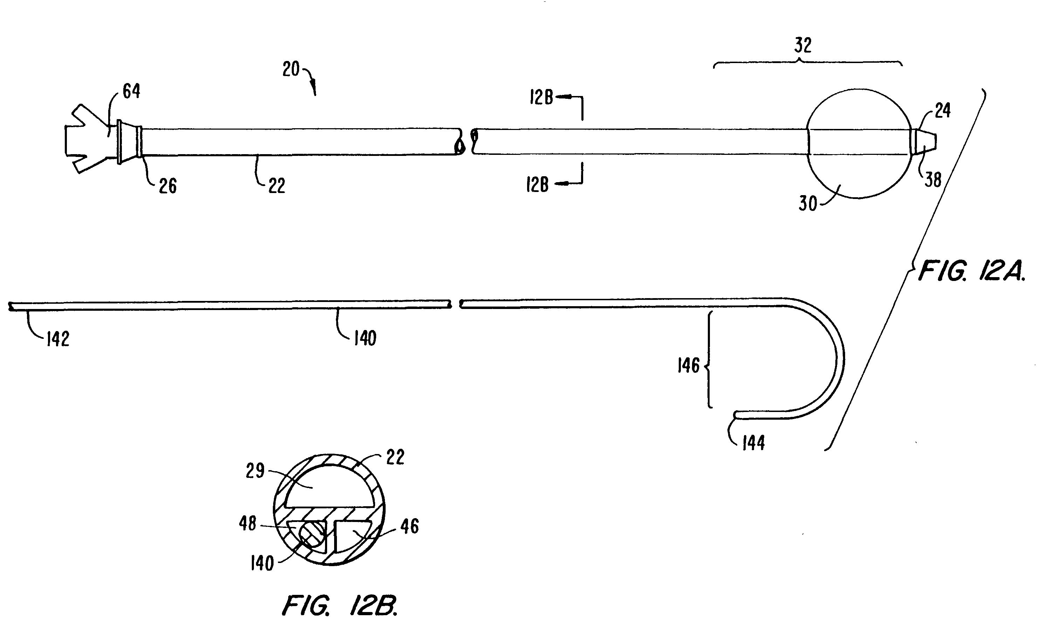

In yet another embodiment, shown in Figures 12A-12B, partitioning device 20 includes a shaping element 140 positionable in a lumen in shaft 22, such as third inner lumen 48. Shaping element 140 has a proximal end 142, a distal end 144 and a preshaped distal portion 146. Preshaped distal portion 146 may be generally U-shaped as illustrated, or may have an angular, "S"-shaped or other configuration in an unstressed condition, which will shape distal portion 32 to generally conform to at least a portion of the patient's aortic arch. Shaping element 140 is preferably stainless steel, nickel titanium alloy, or other biocompatible material with a bending stiffness greater than that of shaft 22 so as to deflect distal portion 32 into the desired shape. Shaping element 140 may be a guidewire over which shaft 22 is advanced to the ascending aorta, or a stylet which is inserted into third inner lumen 48 after shaft 22 is positioned with balloon 30 in the ascending aorta. In a preferred embodiment, shaping element 140 is configured to position distal end 24 of shaft 22 in a radial position within the ascending aorta to be spaced apart from the interior wall thereof, and in particular, axially aligned with the center of the aortic valve.

In a further aspect of the invention, illustrated in Figures 13A-13E, partitioning device 20 is coupled to an arterial bypass cannula 150. Arterial bypass cannula 150 is configured for connection to a cardiopulmonary bypass system for delivering oxygenated blood to the patient's arterial system. Arterial bypass cannula 150 has a distal end 152, a proximal end 154, a blood flow lumen 156 extending between proximal end 154 and distal end 152, and an outflow port 158 at distal end 152. A plurality of additional outflow ports 160 may be provided along the length of arterial bypass cannula 150, particularly near distal end 152. In a preferred embodiment, arterial bypass cannula 150 has a length between about 10 cm and 60 cm, and preferably between about 15 cm and 30 cm.

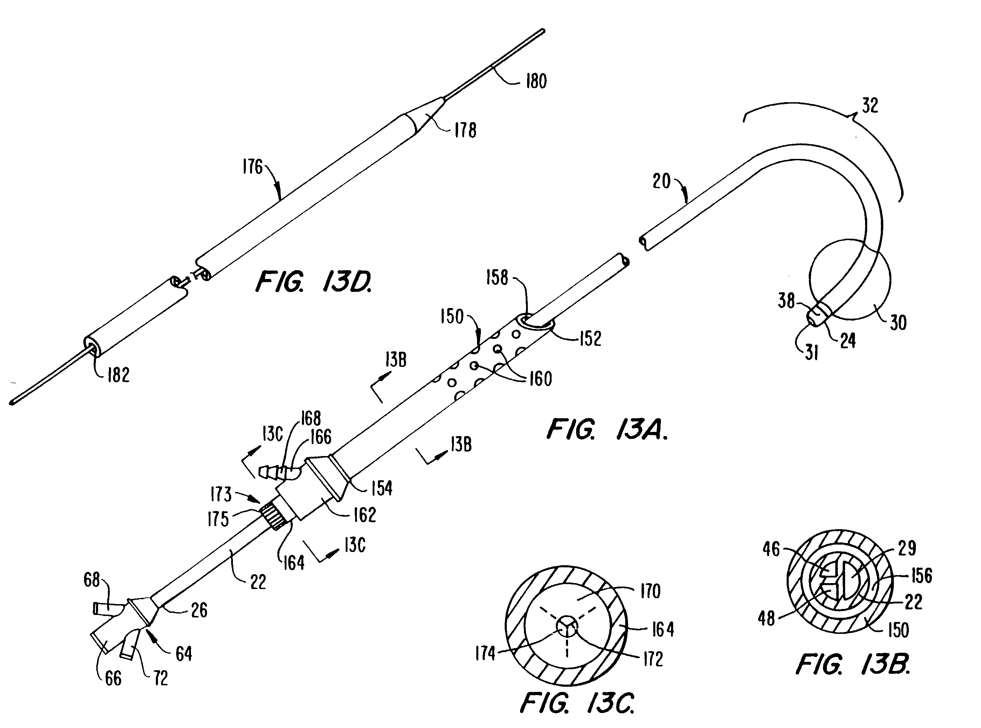

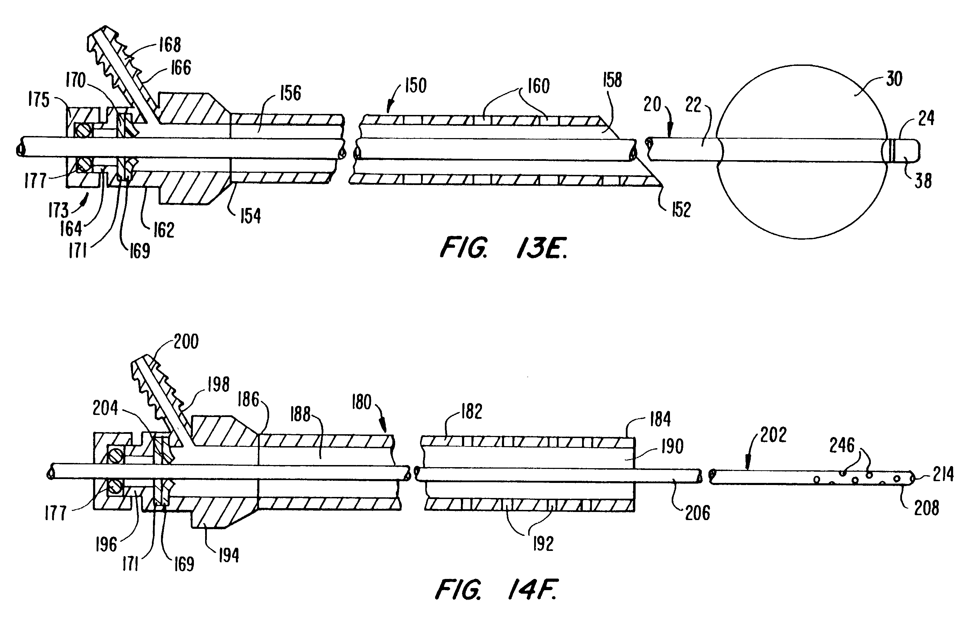

An adaptor 162 is connected to proximal end 154 of bypass cannula 150, and includes a first access port 164 and a second access port 166, both in fluid communication with blood flow lumen 156. Access port 166 is configured for fluid connection to tubing from a cardiopulmonary bypass system, and preferably has a barbed fitting 168. Access port 164 is configured to receive partitioning device 20 therethrough. Preferably, a hemostasis valve 170, shown in Figures 13C and 13E, is mounted in access port 164 to prevent leakage of blood and other fluids through access port 164 whether or not shaft 22 of partitioning device 20 is positioned therein. Hemostasis valve 170 may have any number of well-known constructions, including, for example, an elastomeric disk 169 having one or more slits 172 through which shaft 22 may be positioned, and a diaphragm 171 adjacent to the disk with a central hole 174 for sealing around the periphery of shaft 22. A hemostasis valve of this type is described in US-A-4,000,739. Other types of hemostasis valves may also be used, such as duck-bill valves, O-ring seals, and rotational or sliding mechanical valves. In addition, a Touhy-Borst valve 173 including a threaded, rotatable cap 175 may be provided on the proximal end of access port 164 to facilitate clamping and sealing around shaft 22 by tightening cap 175, which compresses O-rings 177 about shaft 22.

Shaft 22 of partitioning device 20 and blood flow lumen 156 of bypass cannula 150 are configured and dimensioned to facilitate sufficient blood flow through blood flow lumen 156 to support full cardiopulmonary bypass with complete cessation of cardiac activity, without an undesirable level of hemolysis. In a preferred embodiment, arterial bypass cannula 150 has an outer diameter of 6 mm to 10 mm, and blood flow lumen 156 has an inner diameter of 5 mm to 9 mm. Shaft 22 of partitioning device 20 has an outer diameter in the range of 2 mm to 5 mm. In this way, blood flow lumen 156, with shaft 22 positioned therein, facilitates a blood flow rate of at least about 4 liters/minute at a pressure of less than about 250 mmHg.

Arterial bypass cannula 150 is preferably introduced into an artery, usually a femoral artery, with partitioning device 20 removed from blood flow lumen 156. An obturator 176, illustrated in Figure 13D, may be positioned in blood flow lumen 156 such that the tapered distal end 178 of obturator 176 extends distally from the distal end 152 of arterial bypass cannula 150. The arterial bypass cannula 150 may be introduced into the artery by various techniques including percutaneous methods such as the Seldinger technique, but is usually of sufficient size to require a surgical cutdown. A guidewire 180 may be slidably positioned through a lumen 182 in obturator 176 to facilitate introduction of arterial bypass cannula 150. Guidewire 180 is advanced into the artery through an arteriotomy, and arterial bypass cannula 150 with obturator 176 positioned therein is advanced into the artery over guidewire 180. Obturator 176 may then be removed, allowing partitioning device 20 to be introduced into the artery through blood flow lumen 156, usually over guidewire 180. Guidewire 180 may be advanced toward the heart and into the ascending aorta to facilitate positioning the distal end 24 of partitioning device 20 therein.

In an alternative embodiment, arterial bypass cannula 150 may be configured so that partitioning device 20 is not removable from blood flow lumen 156. In this embodiment, bypass cannula 150 is introduced into an artery with partitioning device 20 positioned in blood flow lumen 156. Partitioning device 20 may be slidable within a limited range of movement within blood flow lumen 156. Alternatively, partitioning device 20 may be fixed to arterial bypass cannula 150 to prevent relative movement between the two. For example, shaft 22 may be extruded from the same tubing which is used to form arterial bypass cannula 150. Or, shaft 22 may be attached within the interior of blood flow lumen 156 or at the distal end 152 of arterial bypass cannula 150. Additionally, distal end 152 of bypass cannula 150 may be tapered to seal around shaft 22 and may or may not be bonded to shaft 22. In this configuration, side ports 160 permit outflow of blood from blood flow lumen 156.

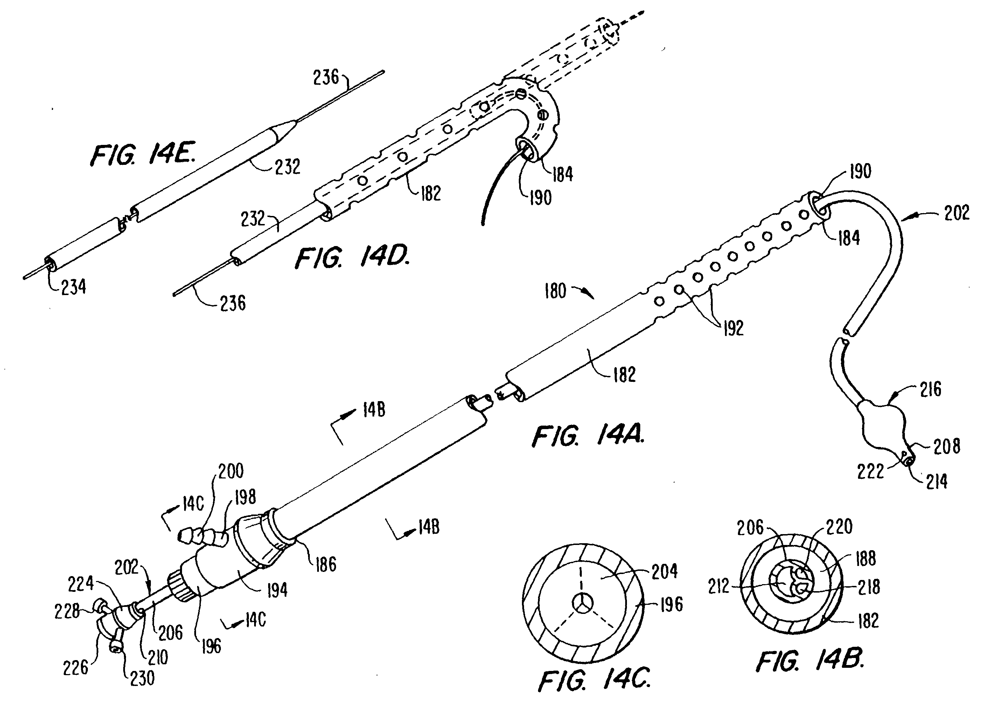

Venting catheter 202 includes an elongated flexible shaft 206 having a distal end 208 and a proximal end 210. An inner lumen 212, shown in Figures 14B and 14F, extends from proximal end 210 to distal end 208, and is in fluid communication with an inflow port 214 in distal end 208. Additional side inflow ports as shown in Figure 14F may also be provided near distal end 208. In one embodiment, an inflatable balloon 216 may be provided near distal end 208 proximal to distal port 214. An inflation lumen 218 extending through shaft 206 is in fluid communication with the interior of balloon 216 for delivering an inflation fluid thereto. Balloon 216 may be used to facilitate placement in the pulmonary artery, to facilitate measurement of wedge pressure in the pulmonary artery, or for other purposes. Additionally, a pressure lumen 220 may be provided in shaft 206, with a pressure port 222 at distal end 208 in fluid communication with pressure lumen 220. This facilitates pressure sensing at distal end 208. A triple arm adaptor 224 is mounted to proximal end 210 of shaft 206. Adaptor 224 has a first access port 226 in fluid communication with inner lumen 212, a second access port 228 in fluid communication with balloon inflation lumen 218, and a third access port 230 in fluid communication with pressure lumen 220.

Blood flow lumen 188 and shaft 206 are dimensioned and configured to facilitate adequate blood flow through blood flow lumen 188 to support full cardiopulmonary bypass with complete cessation of cardiac activity, without an undesirable level of hemolysis. In a preferred embodiment, venous bypass cannula 182 has an outer diameter of 6 mm to 12 mm, while blood flow lumen 188 has an inner diameter of 5 mm to 11.5 mm. Shaft 206 of venting catheter 202 preferably has an outer diameter between about 3 mm and 4 mm. Such a configuration facilitates a blood flow rate of at least about 4 liters/minute at a negative pressure of less than about 20 kPa (150 mmHg).

The distal portion of venous bypass cannula 182 may be straight as shown in Figure 14A, or, alternatively, may have a pre-shaped curvature as shown in Figure 14D. Such a curved configuration may be advantageous in order to guide venting catheter 202 from the right atrium into the right ventricle through the tricuspid valve, as described more fully below. A variety of curves, from a 180° semi-circle, as shown in Figure 14D, to a curve of 90° or less may be provided, according to the direction in which it is desired to guide venting catheter 202. An obturator 232 may be provided for straightening the distal portion for introduction of venous bypass cannula 182. Obturator 232 has a stiffness which is greater than that of the distal portion of venous bypass cannula 182 such that positioning obturator 232 in blood flow lumen 188 straightens the distal portion of bypass cannula 182. Obturator 232 may be provided with an inner lumen 234 through which a movable guidewire 236 may be positioned to facilitate introduction into the patient's venous system.

Cardiac venting device 180 may be introduced using various techniques, but, as with arterial bypass cannula 150 described above, will ordinarily require a surgical cutdown. Usually, venous bypass cannula 182 is introduced into a vein, preferably a femoral vein or internal jugular vein, without venting catheter 202 positioned in blood flow lumen 188. Obturator 232 may be positioned within blood flow lumen 188 to facilitate introduction. Preferably, venous bypass cannula 182 has a length of at least about 75 cm to allow the distal end 184 to be positioned near or within the right atrium of the heart via the inferior vena cava from a femoral vein. Alternatively, venous bypass cannula 182 may have a length of about 50 cm to 70 cm to facilitate introduction through the internal jugular vein in the patient's neck and positioning of distal end 184 in the superior vena cava and/or right atrium. Once venous bypass cannula 182 is in position, venting catheter 202 may be introduced through access port 196 and blood flow lumen 188 until distal end 208 is within the patient's heart. Venting catheter 202 may then be advanced until distal end 208 is in the desired portion of the heart to withdraw blood therefrom. Venting catheter 202 preferably has a length of at least about 110 cm to reach from a femoral vein to the pulmonary artery, or a length of about 70 cm to 90 cm to reach from the internal jugular vein to the pulmonary artery.

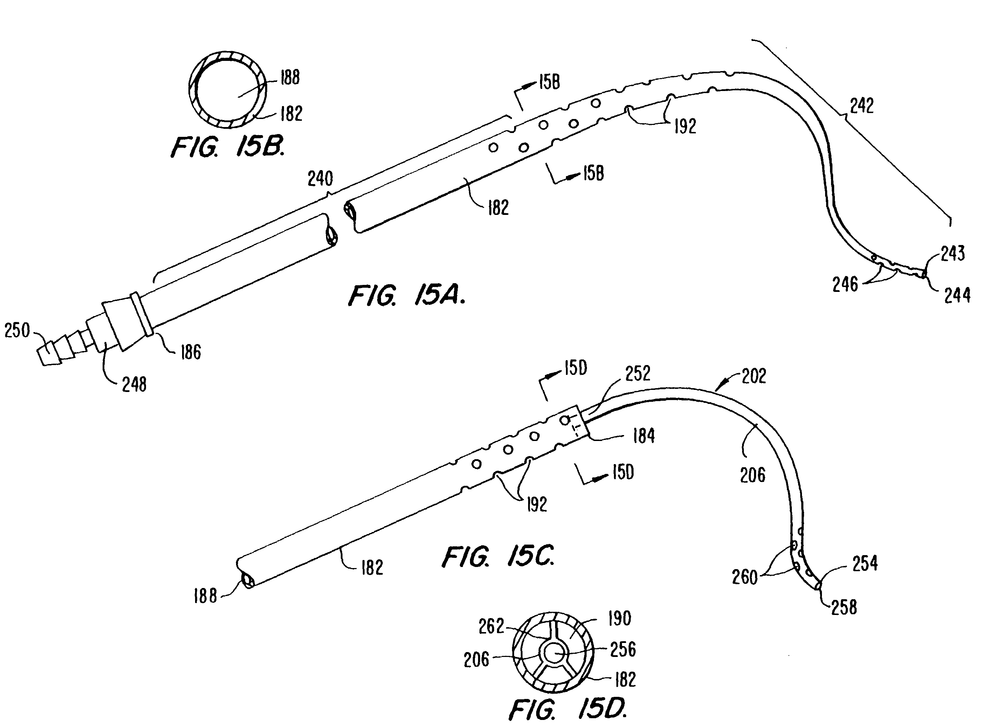

Alternative embodiments of cardiac venting device 180 are illustrated in Figures 15A-15D. In the embodiment of Figure 15A, venous bypass cannula 182 comprises a non-tapered proximal portion 240 and a tapered distal portion 242. Blood flow lumen 188 extends from proximal end 186 to distal end 243. Inflow ports 192 are in fluid communication with blood flow lumen 188 as above. Non-tapered proximal portion 240 preferably has a length selected to allow inflow ports 192 to be positioned within the right atrium of the heart or in the inferior vena cava near the heart. A distal inflow port 244 and side inflow ports 246 are provided at the distal end 243. Distal inflow port 244 and side inflow ports 246 are also in fluid communication with blood flow lumen 188. Additional side inflow ports may be provided over the entire length of tapered section 242. A balloon (not shown) may also be provided at distal end 243, along with a pressure port (not shown), and associated lumens, as provided in previous embodiments. An adaptor 248 is attached to proximal end 186. Adaptor 248 may include an arm 250, preferably having a barbed fitting for connection to a tube from a cardiopulmonary bypass system. Other access ports may be provided in adapter 248 for balloon inflation and pressure measurement.

The total length of venous bypass cannula 182, including proximal portion 240 and tapered distal portion 242, is preferably at least 110 cm to reach the pulmonary artery from a femoral vein, or at least about 70 cm to 90 cm to reach the pulmonary artery from the internal jugular vein.

Tapered portion 242 may be tapered from an outer diameter of 6 mm - 11 mm to an outer diameter of 3 mm - 5 mm at distal end 243, so as to provide the flexibility and small profile necessary for positioning distal end 243 within the pulmonary artery, while maintaining a sufficiently large blood flow lumen 188 to support full cardiopulmonary bypass with cardiac function arrested.

In yet another embodiment, illustrated in Figures 15C and 15D, a shaft 206 of venting catheter 202 has a proximal end 252 which is attached to distal end 184 of venous bypass cannula 182. Shaft 206 has a distal end 254, an inner lumen 256 (Figure 15D), and a distal port 258 in fluid communication with inner lumen 256 at distal end 254. A plurality of additional ports 260 may be provided along shaft 206 near distal end 254. Proximal end 252 of shaft 206 is attached to venous

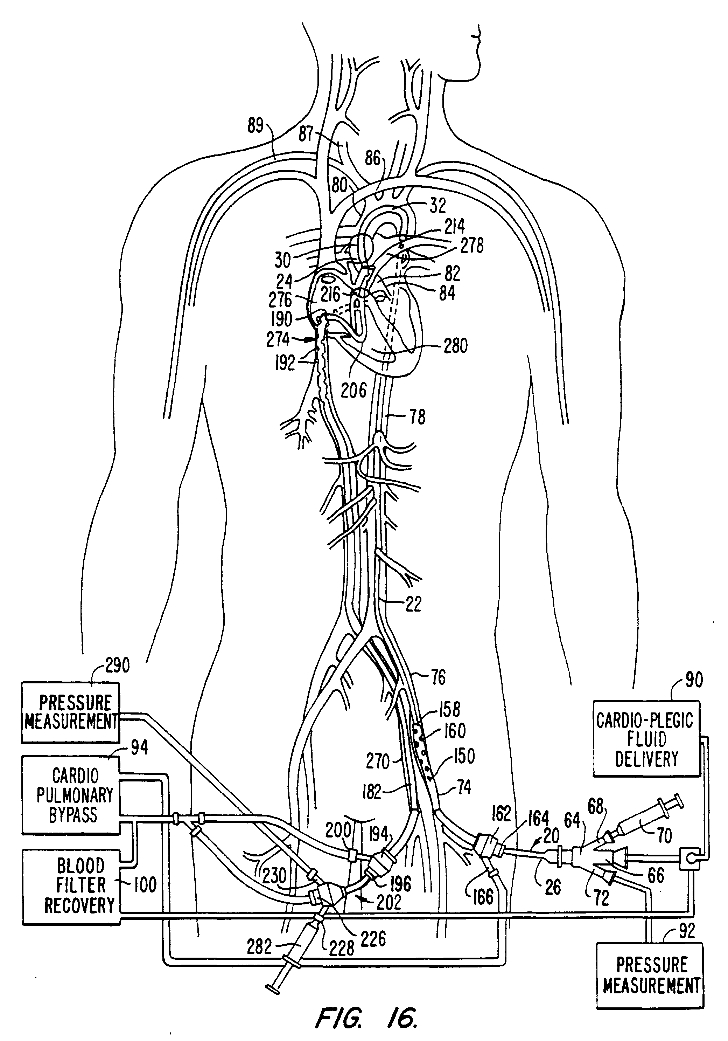

Referring now to Figure 16, the use of the devices illustrated in Figures 13-15 will be described. Arterial bypass cannula 150 is positioned in femoral artery 74, usually by cutdown, with obturator 176 positioned in blood flow lumen 156. Guidewire 180 is first advanced through an arteriotomy into femoral artery 74, and arterial bypass cannula 150 along with obturator 176 are advanced over guidewire 180 into the artery. Obturator 176 may then be removed from blood flow lumen 156. Access port 166 on adaptor 162 is connected to the oxygenated blood outlet of cardiopulmonary bypass system 94.

Cardiopulmonary bypass may then be initiated. Cardiopulmonary bypass system 94 receives deoxygenated blood from the patient's venous system through blood flow lumen 188 of venous bypass cannula 180, oxygenates the blood, and returns the oxygenated blood to blood flow lumen 156 of arterial bypass cannula 150.

Venting catheter 202 is then introduced through access port 196 into blood flow lumen 188. Venting catheter 202 is advanced toward the heart through blood flow lumen 188, and through distal port 190 into the right atrium 276. The venting catheter may be positioned in various locations within the heart, however, in a preferred embodiment, venting catheter 202 is positioned such that distal port 214 is within the pulmonary artery 278. Usually, this will be accomplished by positioning a Swan-Ganz catheter through blood flow lumen 188 and into right atrium 276 before introducing venting catheter 202. Usually, a balloon on the distal end of the Swan-Ganz catheter is inflated within the right atrium, and the distal end of the Swan-Ganz catheter is advanced from the right atrium 276, through the right ventricle 280, and into the pulmonary artery 278. Once the Swan-Ganz catheter has been positioned in the pulmonary artery, the balloon at its distal end may be deflated, and venting catheter 202 is advanced over the Swan-Ganz catheter until the distal end 208 of venting catheter 202 is within the pulmonary artery. The Swan-Ganz catheter may then be removed from the patient.

Access port 226 at the proximal end of venting catheter 202 is connected to a deoxygenated blood inlet of cardiopulmonary bypass system 94. Venting catheter 202 withdraws blood from the pulmonary artery 278 and delivers the blood to cardiopulmonary bypass system 94. Alternatively, access port 226 may be connected to a separate roller pump (not shown) which feeds the blood withdrawn from the heart into filter/recovery reservoir 100, then returns the blood to CPB system 94. If a balloon 216 is provided at the distal end of venting catheter 202, a balloon inflation device, such as a syringe 282, is connected to access port 228, and inflation fluid is injected into balloon 216. A pressure

It will be understood to those of skill in the art that a variety of devices may be introduced through blood flow lumen 156 of arterial bypass cannula 150 in addition to aortic partitioning device 22. For example, coronary angioplasty or atherectomy catheters may be introduced through arterial bypass cannula 150 and advanced into the coronary arteries, facilitating CPB assist during angioplasty and atherectomy procedures through a single femoral arterial penetration. Electrophysiology catheters for myocardial mapping and ablation may be introduced through arterial bypass cannula 150 and advanced into the heart or coronary arteries to facilitate CPB assist during such procedures without an additional femoral arterial or venous penetration. A variety of endovascular instruments for inspecting and treating the heart and great vessels, including angioscopes, valve repair devices, valve removal devices, devices for introduction and attachment of valve prostheses, septal defect repair devices, aneurysm treatment devices, and others may be introduced through arterial bypass cannula 150, facilitating CPB assist during such interventional procedures without requiring additional arterial or venous penetrations.

The device disclosed herein offers significant advantages over conventional techniques. Important among these advantages is the ability to establish cardiopulmonary bypass and perform interventional procedures within the heart and great vessels with a minimum of venous and arterial penetrations, thereby reducing substantially the morbidity and mortality of such procedures. Further, the invention facilitates performing such interventional procedures and establishing cardiopulmonary bypass through a single arterial penetration and a single venous penetration. In this way, the invention not only reduces the total number of penetrations and the associated trauma and risks attendant such penetrations, but allows a greater number of patients to receive closed-chest surgical treatment who, because of conditions in one or more femoral vessels, would otherwise be prevented from receiving such treatment.

The invention further facilitates arresting cardiac function and establishing cardiopulmonary bypass by means of an endovascular device introduced through a single femoral arterial penetration, eliminating the need for a conventional gross thoracotomy. By obviating the need to open the chest for external clamping of the aorta, the invention facilitates the performance of a new generation of minimally-invasive cardiac and vascular procedures. Elimination of a gross thoracotomy in such procedures produces lower mortality and morbidity, reduced patient suffering, decreased hospitalization and recovery time, and reduced medical costs. Moreover, the invention is useful even in open-chest procedures as a substitute for the aortic cross-clamp where calcification or other conditions could make external aortic clamping undesirable.

## Claims

1. An endovascular device (20) for partitioning a patient's ascending aorta (82) between the coronary ostia (84) and the brachiocephalic artery (86) and infusing oxygenated blood into a patient's arterial system downstream of the coronary ostia, the device comprising:
a bypass cannula (150) having a distal end (152) configured for introduction into an artery, a proximal end (154), a blood flow lumen (156) therebetween, and an outflow port (158) at the distal end (152) in fluid communication with the blood flow lumen;
an elongated catheter shaft (22) having a distal end (24), a proximal end (26) and an inner lumen (29) therebetween, and a first port (31) at the distal end (24) in fluid communication with the inner lumen (29);
**characterised in that**:
the elongated catheter shaft is positioned within the blood flow lumen (156) of the bypass cannula (150) so as to extend distally from the distal end (152) thereof, and wherein the blood flow lumen (156) is sized to support cardiopulmonary bypass;
the elongate catheter shaft (22) has a shaped distal portion (32);
the distal end (24) of the elongated catheter shaft (22) is configured for positioning in the patient's ascending aorta (82); and
the device (20) further includes expandable means (28) near the distal end (24) of the catheter shaft (22) for occluding the ascending aorta (82) between the coronary ostia (84) and the brachiocephalic artery (86) so as to block substantially all blood flow therethrough.

2. An endovascular device according to claim 1 wherein the catheter shaft (22) is slidably disposed in the blood flow lumen (156) of the bypass cannula (150), the catheter shaft having a cross-sectional profile configured to allow blood flow through the blood flow lumen when the catheter shaft is positioned therein.

3. An endovascular device according to claim 1 wherein the blood flow lumen (156) is configured to facilitate a flow rate of blood of at least about 4 liters/minute at a pressure of less than about 33 kPa (250 mmHg).

4. An endovascular device according to claim 1 wherein the bypass cannula (150) has an outer diameter of less than about 10 mm.

5. An endovascular device according to claim 1 further comprising an adaptor (162) assembly connected to the proximal end (154) of the bypass cannula (150), the adaptor assembly having first (164) and second access ports (166) in fluid communication with the blood flow lumen (156), the first access port being configured to receive the catheter shaft (22), and the second access port being configured for connection to an oxygenated blood supply.

6. An endovascular device according to claim 5 wherein the first access port (164) includes a hemostasis valve (170) for fluidly sealing about the catheter shaft (22).

7. An endovascular device according to claim 1 wherein the catheter shaft (22) has a length of at least about 80 cm to facilitate transluminal positioning from a femoral artery to the ascending aorta (82).

8. An endovascular device according to claim 7 wherein the bypass cannula (150) has a length between about 10 cm and 60 cm.

9. An endovascular device according to claim 1 wherein the expandable means (28) comprises an inflatable balloon (30) having an interior for receiving an inflation fluid, the catheter shaft further comprising an inflation lumen (58) in fluid communication with the interior of the balloon for delivering an inflation fluid into the balloon.

10. An endovascular device according to claim 1 further comprising a pressure lumen (48) extending within the catheter shaft (22) from the proximal end (26) to the distal end (24) thereof, and a second port (72) at the distal end in communication with the pressure lumen for sensing pressure upstream of the occluding means.

11. An endovascular device according to claim 1 wherein the cather shaft (22) is removable from the bypass cannula (150).

12. A system for arresting a patient's heart and delivering oxygenated blood to the patient's arterial system, the system comprising:
an endovascular device (20) according to claim 1;
means at the proximal end (154) of the bypass cannula (150) in fluid communication with the blood flow lumen (156) for delivering oxygenated blood into the arterial system downstream of the expanding means (28); and
means for paralysing the patient's myocardium.

13. A system according to claim 12 wherein the means for paralysing the patient's myocardium comprises a fluid delivery device (90) in communication with the inner lumen (29) for delivering cardioplegic fluid upstream of the expanding means (28).

14. A system according to claim 12 wherein the means for delivering oxygenated blood comprises a cardiopulmonary bypass system (94) including means (96) for receiving blood from a venous location in the patient, means for oxygenating the blood, and means for delivering the oxygenated blood to the blood flow lumen (156).

## Patentansprüche

1. Endovaskuläre Vorrichtung (20) zum Teilen der aufsteigenden Aorta (82) eines Patienten zwischen dem Koronarostium (84) und der Brachiozephalarterie (86) und Infundieren von arterialisiertem Blut in das Arteriensystem eines Patienten stromab vom Koronarostium, wobei die Vorrichtung umfasst:
eine Bypass-Kanüle (150) mit einem distalen Ende (152), das zum Einführen in eine Arterie konfiguriert ist, einem proximalen Ende (154), einem Blutstromlumen (156) dazwischen und einer Ausflussöffnung (158) am distalen Ende (152) in Fluidkommunikation mit dem Blutstromlumen;
einen länglichen Katheterschaft (22) mit einem distalen Ende (24), einem proximalen Ende (26) und einem inneren Lumen (29) dazwischen, sowie einer ersten Öffnung (31) am distalen Ende (24) in Fluidkommunikation mit dem inneren Lumen (29);
**dadurch gekennzeichnet, dass**
der längliche Katheterschaft so innerhalb des Blutstromlumens (156) der Bypass-Kanüle (150) angeordnet ist, dass er sich distal von ihrem distalen Ende (152) weg erstreckt, und worin das Blutstromlumen (156) eine solche Größe hat, dass sie pneumokardialen Bypass stützt;
der längliche Katheterschaft (22) einen geformten distalen Abschnitt (32) aufweist;
das distale Ende (24) des länglichen Katheterschafts (22) eine solche Konfiguration aufweist, um es in der aufsteigenden Aorta (82) des Patienten anzuordnen; und
die Vorrichtung (20) weiters erweiterbare Mittel (28) nahe dem distalen Ende (24) des Katheterschafts (22) umfasst, um die aufsteigende Aorta (82) zwischen dem Koronarostium (84) und der Brachiozephalarterie (86) zu verschließen, um im Wesentlichen den gesamten Blutstrom durch sie hindurch zu blockieren.

2. Endovaskuläre Vorrichtung nach Anspruch 1, worin der Katheterschaft (22) gleitend im Blutstromlumen (156) der Bypass-Kanüle (150) angeordnet ist, wobei der Katheterschaft ein Querschnittsprofil aufweist, das eine Konfiguration aufweist, die das Strömen von Blut durch das Blutstromlumen zulässt, wenn der Katheterschaft darin angeordnet ist.

3. Endovaskuläre Vorrichtung nach Anspruch 1, worin das Blutstromlumen (156) eine Konfiguration aufweist, die eine Blutströmungsrate von zumindest etwa 4 l/min bei einem Druck von weniger als etwa 33 kPa (250 mmHg) erleichtert.

4. Endovaskuläre Vorrichtung nach Anspruch 1, worin die Bypass-Kanüle (150) einen Außendurchmesser von weniger als etwa 10 mm aufweist.

5. Endovaskuläre Vorrichtung nach Anspruch 1, weiters umfassend eine Adapteranordnung (162), die mit dem proximalen Ende (154) der Bypass-Kanüle (150) verbunden ist, wobei die Adapteranordnung eine erste (164) und eine zweite Zugangsöffnung (166) in Fluidkommunikation mit dem Blutstromlumen (156) aufweist, wobei die erste Zugangsöffnung eine Konfiguration aufweist, um den Katheterschaft (22) aufzunehmen, und die zweite Zugangsöffnung eine Konfiguration zum Anschließen an eine Zufuhr für arterialisiertes Blut aufweist.

6. Endovaskuläre Vorrichtung nach Anspruch 5, worin die erste Zugangsöffnung (164) ein Hämostase-Ventil (170) zur Fluidabdichtung um den Katheterschaft (22) umfasst.

7. Endovaskuläre Vorrichtung nach Anspruch 1, worin der Katheterschaft (22) eine Länge von zumindest etwa 80 cm aufweist, um die transluminale Positionierung von einer Oberschenkelarterie weg zur aufsteigenden Aorta (82) zu erleichtern.

8. Endovaskuläre Vorrichtung nach Anspruch 7, worin die Bypass-Kanüle (150) eine Länge zwischen etwa 10 cm und 60 cm aufweist.

9. Endovaskuläre Vorrichtung nach Anspruch 1, worin das erweiterbare Mittel (28) einen aufblasbaren Ballon (30) umfasst, der einen Innenraum zum Aufnehmen eines Aufblasfluids aufweist, wobei der Katheterschaft weiters ein Aufblaslumen (58) in Fluidkommunikation mit dem Innenraum des Ballons umfasst, um Aufblasfluid in den Ballon zuzuführen.

10. Endovaskuläre Vorrichtung nach Anspruch 1, weiters umfassend ein Drucklumen(48), das sich innerhalb des Katheterschafts (22) von seinem proximalen Ende (26) zu seinem distalen Ende (24) erstreckt, sowie eine zweite Öffnung (72) am distalen Ende in Kommunikation mit dem Drucklumen, um den Druck stromauf vom Verschlussmittel zu messen.

11. Endovaskuläre Vorrichtung nach Anspruch 1, worin der Katheterschaft (22) von der Bypass-Kanüle (150) abnehmbar ist.

12. System, um das Herz eines Patienten zum Stillstand zu bringen und dem Arteriensystem des Patient arterialisiertes Blut zuzuführen, wobei das System Folgendes umfasst:
eine endovaskuläre Vorrichtung (20) nach Anspruch 1,
Mittel am proximalen Ende (154) der Bypass-Kanüle (150) in Fluidkommunikation mit dem Blutstromlumen (156), um arterialisiertes Blut in das Arteriensystem stromab vom Erweiterungsmittel (28) abzugeben; und
Mittel zum Paralysieren des Herzmuskels des Patienten.

13. System nach Anspruch 12, worin das Mittel zum Paralysieren des Herzmuskels des Patienten eine Fluidabgabevorrichtung (90) in Kommunikation mit dem Innenlumen (29) umfasst, um stromauf vom Erweiterungsmittel (28) kardioplegisches Fluid zuzuführen.

14. System nach Anspruch 12, worin das Mittel zum Zuführen von arterialisiertem Blut ein pneumokardiales Bypass-System (94) umfasst, das Mittel (96) zum Aufnehmen von Blut von einer venösen Stelle im Patienten, Mittel zum Arterialisieren des Bluts und Mittel zum Abgeben des arterialisierten Bluts an das Blutstromlumen (156) umfasst.

## Revendications

1. Dispositif endovasculaire (20) pour séparer l'aorte ascendante (82) d'un patient entre les orifices coronaires (84) et le tronc artériel brachio-céphalique (86) et pour infuser du sang oxygéné dans le système artériel d'un patient en aval des orifices coronaires, le dispositif comprenant:
une canule de circulation extracorporelle (150) présentant une extrémité distale (152) configurée pour l'introduction dans une artère, une extrémité proximale (154), une lumière d'écoulement de sang (156) entre celles-ci et un orifice de sortie (158) à l'extrémité distale (152) en communication de fluide avec la lumière d'écoulement de sang;
une tige de cathéter oblongue (22) ayant une extrémité distale (24), une extrémité proximale (26) et une lumière intérieure (29) entre celles-ci, et un premier orifice (31) à l'extrémité distale (24) en communication de fluide avec la lumière intérieure (29);
**caractérisé en ce que**:
la tige de cathéter oblongue est positionnée dans la lumière (156) de l'écoulement de sang de la canule de circulation extracorporelle (150) de manière à s'étendre distalement à partir de l'extrémité distale (152) de celle-ci, et où la lumière (156) de l'écoulement de sang est dimensionnée pour supporter la circulation extracorporelle;
la tige de cathéter oblongue (22) présente une portion distale configurée (32) ;
l'extrémité distale (24) de la tige de cathéter oblongue (22) est configurée pour être positionnée dans l'aorte ascendante (82) du patient; et
le dispositif (20) comprend en outre des moyens expansibles (28) près de l'extrémité distale (24) de la tige de cathéter (22) pour fermer l'aorte ascendante (82) entre les orifices coronaires (84) et le tronc artériel brachio-céphalique (86) de manière à bloquer sensiblement tout écoulement de sang à travers celle-ci.

2. Dispositif endovasculaire selon la revendication 1, où la tige de cathéter (22) est disposée d'une manière coulissante dans la lumière (156) de l'écoulement de sang de la canule de circulation extracorporelle (150), la tige de cathéter ayant un profil en section transversal configuré pour permettre un écoulement du sang à travers la lumière d'écoulement de sang lorsque la tige de cathéter est positionnée dans celle-ci.

3. Dispositif endovasculaire selon la revendication 1, où la lumière (156) de l'écoulement de sang est configurée pour faciliter un débit d'écoulement de sang d'au moins environ 4 litres/minute à une pression inférieure à environ 33 KPa (250 mmHg).

4. Dispositif endovasculaire selon la revendication 1, où la canule de circulation extracorporelle (150) a un diamètre extérieur inférieur à environ 10 mm.

5. Dispositif endovasculaire selon la revendication 1, comprenant en outre un ensemble formant adaptateur (162) relié à l'extrémité proximale (154) de la canule de circulation extracorporelle (150), l'ensemble formant adaptateur ayant des premier (164) et second (166) orifices d'accès en communication de fluide avec la lumière (156) d'écoulement de sang, le premier orifice d'accès étant configuré pour recevoir la tige de cathéter (22), et le second orifice d'accès étant configuré pour la connexion à une alimentation de sang oxygéné.

6. Dispositif endovasculaire selon la revendication 5, où le premier orifice d'accès (164) comprend une vanne d'hémostase (170) en vue d'une étanchéité au fluide autour de la tige de cathéter (22).

7. Dispositif endovasculaire selon la revendication 1, où la tige de cathéter (22) a une longueur d'au moins environ 80 cm pour faciliter un positionnement transluminal d'une artère fémorale à l'aorte ascendante (82).

8. Dispositif endovasculaire selon la revendication 7, où la canule de circulation extracorporelle (150) a une longueur entre environ 10 cm et 60 cm.

9. Dispositif endovasculaire selon la revendication 1, où le moyen expansible (28) comprend un ballon gonflable (30) dont l'intérieur est prévu pour recevoir un fluide de gonflement, la tige de cathéter comprenant en outre une lumière de gonflement (58) en communication de fluide avec l'intérieur du ballon pour introduire un fluide d'inflation dans le ballon.

10. Dispositif endovasculaire selon la revendication 1, comprenant en outre une lumière de pression (48) s'étendant dans la tige de cathéter (22) à partir de l'extrémité proximale (26) à l'extrémité distale (24) de celle-ci, et un second orifice (72) à l'extrémité distale en communication avec la lumière de pression pour détecter la pression en amont du moyen d'occlusion.

11. Dispositif endovasculaire selon la revendication 1, où la tige de cathéter (22) peut être retirée de la canule de circulation extracorporelle (150).

12. Système pour arrêter le coeur d'un patient et pour transmettre du sang oxygéné au système artériel du patient, le système comprenant:
un dispositif endovasculaire (20) selon la revendication 1;
des moyens à l'extrémité proximale (154) de la canule de circulation extracorporelle (150) en communication de fluide avec la lumière d'écoulement de sang (156) pour transmettre du sang oxygéné dans le système artériel en aval du moyen d'expansion (28); et
des moyens pour paralyser le myocarde du patient.

13. Système selon la revendication 12, où le moyen pour paralyser le myocarde du patient comprend un dispositif de transmission de fluide (90) en communication avec la lumière interne (29) pour transmettre un fluide cardioplégique en amont du moyen d'expansion (28).

14. Système selon la revendication 12, où le moyen pour transmettre du sang oxygéné comprend un système de circulation extracorporelle (94) incluant des moyens (96) pour recevoir du sang d'un emplacement veineux dans le patient, des moyens pour oxygéner le sang et des moyens pour transmettre le sang oxygéné à la lumière d'écoulement de sang (156).
